Europäisches Patentamt
European Patent Office
Office européen des brevets

(19) (11) Publication number: **0 052 361 A1**

# (12) EUROPEAN PATENT APPLICATION

(21) Application number: 81109685.8

(22) Date of filing: 13.11.81

(51) Int. Cl.³: C 07 H 17/08
A 61 K 31/70
//C12P19/62, (C12P19/62,
C12R1/54)

(30) Priority: 18.11.80 JP 161342/80
14.08.81 JP 126577/81

(43) Date of publication of application:
26.05.82 Bulletin 82/21

(84) Designated Contracting States:
DE FR GB

(71) Applicant: SANRAKU-OCEAN CO., LTD.
15-1, Kyobashi 1-chome Chuo-ku
Tokyo(JP)

(72) Inventor: Okamoto, Rokuro
5437, Fujisawa
Fujisawa-shi Kanagawa-ken(JP)

(72) Inventor: Yamamoto, Masao
4514-6, Fujisawa
Fujisawa-shi Kanagawa-ken(JP)

(72) Inventor: Takada, Kazuaki
15-609, Takamura Danchi 203, Takamura
Hiratsuka-shi Kanagawa-ken(JP)

(72) Inventor: Kiyoshima, Kohki
Sanraku-ryo, 555 Nagata-cho Minami-ku
Yokohama-shi Kanagawa-ken(JP)

(72) Inventor: Ishikura, Tomoyuki
22-32 Kowada 1-chome
Chigasaki-shi Kanagawa-ken(JP)

(72) Inventor: Umezawa, Hamao
23, Toyotamakita 4-chome
Nerima-ku Tokyo(JP)

(72) Inventor: Takeuchi, Tomio
5-1-11, Higashigotanda
Shinagawa-ku Tokyo(JP)

(74) Representative: Kraus, Walter, Dr. et al,
Patentanwälte Dres. Kraus & Weisert Irmgardstrasse 15
D-8000 München 71(DE)

## (54) Novel 16-membered macrolide compounds.

(57) A compound of the general formula

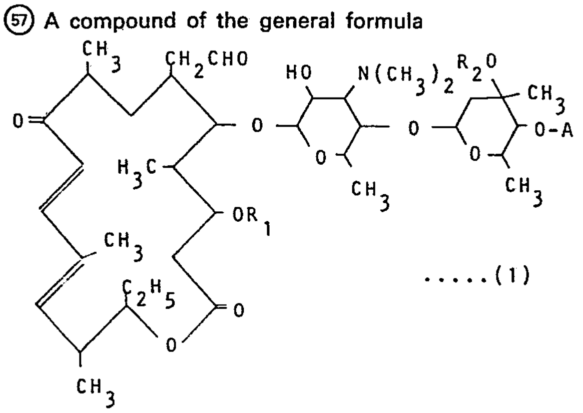

.....(1)

wherein $R_1$ and $R_2$, independently from each other, represent a hydrogen atom or a lower alkanoyl group; and A represents a hydrogen atom or a group of the formula $-Y-R_3$ in which $R_3$ represents an alkyl group, a cycloalkyl group, an aryl group, an aralkyl group, a 4- to 7-membered monovalent heterocyclic group containing 1 to 4 hetero atoms selected from nitrogen, oxygen and sulfur atoms or a group of the formula $-R_4-Z-R_5$ in which $R_4$ represents a lower alkylene group which may be substituted by a hydroxyl or lower alkanoyloxy group, $R_5$ represents an alkyl group, a cycloalkyl group, an aryl group, an aralkyl group, or a 4- to 7-membered monovalent heterocyclic group containing 1 to 4 hetero atoms selected from nitrogen, oxygen and fulfur atoms and Z represents an oxygen or sulfur atom, and Y represents $-CO-$, $-SO_2-$, or $-CONH-$, or Y and $R_3$, taken together, represent a tetrahydrofuranyl or tetrahydropyranyl group; and the aforesaid aryl group, aralkyl group and heterocyclic group may be substituted, as required, by a lower alkyl, lower alkoxy or nitro group; or its salt. This compound is useful as a medicine for the prevention, therapy or treatment of bacterial infections of mammals.

EP 0 052 361 A1

This invention relates to novel macrocyclic compounds, and more specifically, to antibiotic YT-3927 having a 16-membered macrolide structure and its derivatives having excellent antibacterial activity.

Many 16-membered macrolide antibiotics such as tylosin, carbomycin, leucomycin and rosamicin have been known. Tylosin is the oldest type of 16-membered macrolide antibiotics. Because tylosin has low absorbability and excretability in vivo despite its high antibacterial activity in vitro, investigations have been actively undertaken in the art for producing tylosin derivatives having excellent absorbability and excretability by chemical or biological converting methods.

It is well known that Streptomyces fradiae NRRL B-2702 strain produces tylosin (see, for example, Antibiot. & Chemoth., Vol. 11, Pages 328 - 334, 1961).

From quite a different standpoint from prior works, the present inventors hit upon an idea of mutating the aforesaid Streptomyces fradiae NRRL B-2702, a tylosin-producing microorganism, under various conditions in order to search for novel analogs of tylosin. Subsequent investigations based on this idea showed that when the Streptomyces fradiae NRRL B-2702 is mutated by using N-methyl-N'-nitro-N-nitrosoguanidine as a mutagenic agent and the resulting novel mutant, Streptomyces fradiae YT-3927, is cultivated in a nutrient medium, a novel tylosin analog of the following formula

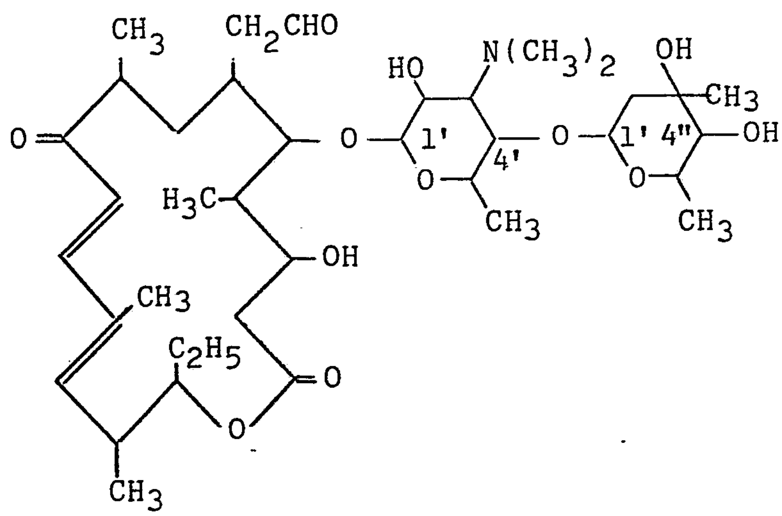

(I-a)

resulting from the substitution of a hydrogen atom for the mycinose residue bonded to the carbon atom at the 23-position in the carbon numbering of tylosin can be obtained from the culture broth, and that this compound exhibits broad and strong antibacterial activity against various pathogenic microorganisms such as Gram-positive bacteria, Gram-negative bacteria, and mycoplasmas.

They named this compound "antibiotic YT-3927".

The antibiotic YT-3927 provided by this invention has a 16-membered macrolide structure as is seen from the above formula (I-a), and is characterized by the bonding of a methyl group at its 14-position. This compound has physicochemical properties shown below.

(1) Appearance: white crystals

(2) Melting point: 164.5 - 168$^{o}$C

(3) Specific rotation: $[\alpha]_D^{25} = -6.6^{o}$ (c=2, methanol)

(4) Elemental analysis for $C_{38}H_{63}NO_{12}$:

| | C | H | N |
|---|---|---|---|
| Calculated (%): | 62.87 | 8.75 | 1.93 |
| Found (%): | 63.03 | 8.80 | 1.84 |

(5) Ultraviolet absorption spectrum:

$\lambda_{max}^{methanol} = 283$ nm ($E_{1\,cm}^{1\%} = 300$)

(6) Infrared absorption spectrum

The main characteristic absorption peaks (wave numbers) of the infrared spectrum measured by the potassium bromide tablet method are as follows:

3470, 2980, 2940, 2720, 1725, 1685, 1600 $cm^{-1}$.

(7) Proton NMR spectrum

The characteristic signals of the proton NMR spectrum in deuterochloroform at 100 MHz and their assignments are as follows (tetramethylsilane is used as an internal reference).

| δ (ppm) | Number of protons | Form | J (Hz) | Assignment |
|---|---|---|---|---|
| 0.95 | 3 | t | $J_{16,17}$ =7.0 | 17-$CH_3$ |
| 1.09 | 3 | d | $J_{14,23}$ =6.8 | 23-$CH_3$ |
| 1.81 | 3 | s | | 22-$CH_3$ |
| 2.49 | 6 | s | | $N(CH_3)_2$ |
| 3.84 | 1 | broad d | $J_{2A,3}$ =10<br>$J_{3,4}$ =about 1-2<br>$J_{2B,3}$ =about 1-2 | 3-H |
| 4.08 | 1 | qd | $J_{5",6"}$ =6.0<br>$J_{4",5"}$ =9.6 | 5"-H |
| 4.23 | 1 | d | $J_{1',2'}$ =7.4 | 1'-H |
| 4.73 | 1 | ddd | $J_{14,15}$ =9.5<br>$J_{15,16A}$=3.0<br>$J_{15,16B}$=9.0 | 15-H |
| 5.08 | 1 | broad d | $J_{1",2"A}$=3.0<br>$J_{1",2"B}$=about 0.5 | 1"-H |
| 5.67 | 1 | d | $J_{13,14}$=10.5 | 13-H |
| 6.28 | 1 | d | $J_{10,11}$=15.3 | 10-H |
| 7.27 | 1 | d | $J_{11,12}$=15.3 | 11-H |
| 9.67 | 1 | d | | CHO |

(8) Molecular weight and molecular formula

The molecular weight of the antibiotic YT-3927 is 725 when determined by an electron impact mass spectrum. The molecular formula is $C_{38}H_{63}NO_{12}$.

(9) Solubility

Soluble in lower alcohol such as methanol and ethanol, esters such as ethyl acetate and butyl acetate, ketones such as acetone and methyl isobutyl ketone, benzene, toluene, chloroform, and weakly acidic water, and insoluble in weakly alkaline water, petroleum ether, n-hexane and cyclohexane.

(10) Stability

Stable even when left to stand at room temperature for one month.

(11) Color reactions

Positive in the Molisch reaction, anthrone reaction and conc. sulfuric acid reaction, and negative in the burette reaction, ninhydrin reaction and Sakaguchi reaction.

(12) $C^{13}$ NMR spectrum

The $C^{13}$ NMR spectrum in deuterochloroform at 25.2 MHz is shown in Figure 1 attached (internal reference: tetramethyl silane).

The above antibiotic YT-3927 exhibits fairly strong activity against various pathogenic microorganisms such as Gram-positive bacteria, Gram-negative bacteria and mycoplasmas. The inventors of the present application, however, furthered their investigations in order to develop tylosin derivatives having much stronger antibacterial activity and higher concentrations in the blood and showing high bioavailability. These further investigations have led to the discovery of novel compounds of the following general formula

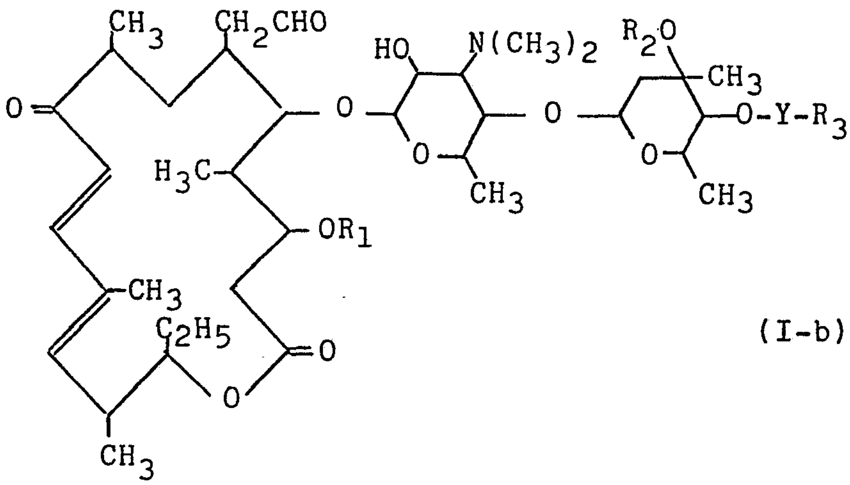

(I-b)

wherein $R_1$ and $R_2$, independently from each other, represent a hydrogen atom or a lower alkanoyl group; $R_3$ represents an alkyl group, a cycloalkyl group, an aryl group, an aralkyl group, a 4- to 7-membered monovalent heterocyclic group containing 1 to 4 hetero atoms selected from nitrogen, oxygen and sulfur atoms, or a group of the formula $-R_4-Z-R_5$ in which $R_4$ represents a lower alkylene group which may be substituted by a hydroxyl or lower alkanoyloxy group, $R_5$ represents an alkyl group, a cycloalkyl group, an aryl group, an aralkyl group or a 4- to 7-membered monovalent heterocyclic group containing 1 to 4 hetero atoms selected from nitrongen, oxygen and sulfur atoms, and Z represents an oxygen or sulfur atom; and Y represents -CO-, $-SO_2-$, or -CONH-, or Y and $R_3$, taken together, represent a tetrahydrofuranyl or tetrahydropyranyl group; provided that the aforesaid aryl, aralkyl and heterocyclic groups may be substituted by a lower alkyl group, a lower alkoxy group or a nitro group; and the salts of said compounds.

Thus, the present invention provides compounds of the following general formula (I) and their salts.

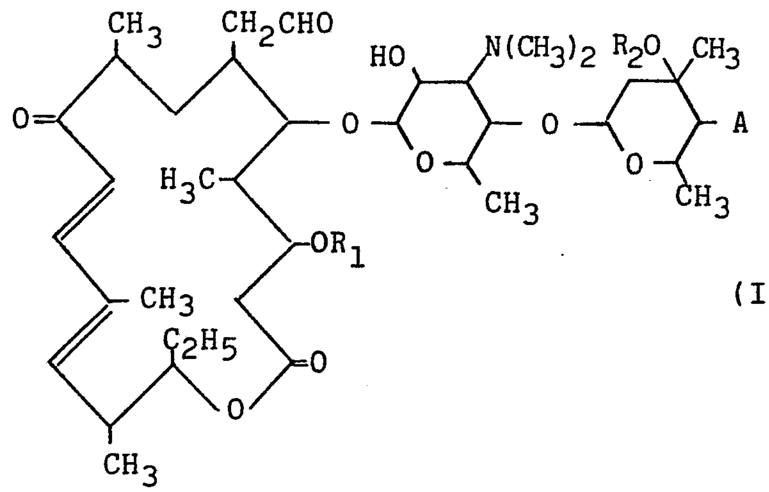

(I)

wherein A represents a hydrogen atom or a group of the formula $-Y-R_3$; and $R_1$, $R_2$, $R_3$ and Y are as defined hereinabove.

The compounds of formula (I) and their salts provided by this invention, a process for their production, and their medical use will be described in greater detail below.

The term "lower", as used in the present specification and the appended claims to qualify groups or compounds, means that the groups or compounds so qualified have not more than 6, preferably not more than 4, carbon atoms.

The term "lower alkanoyl group", used herein, denotes a lower alkylcarbonyl group whose lower alkyl moiety is linear or branched, and includes, for example, acetyl, propionyl, n-butyryl, isobutyryl, n-valeryl, iso-valeryl, and n-caproyl A hydrogen atom or an acetyl group is preferred as the group $R_1$ and/or $R_2$ in formula (I) or (I-b).

The "alkyl group" used in the definition of $R_3$ may be linear or branched, and may generally contain 1 to 6 carbon atoms, preferably 1 to 4 carbon atoms. Examples are methyl, ethyl, n-propyl, isopropyl, n-butyl,

isobutyl, sec-butyl, tert-butyl, n-pentyl, isopentyl, n-hexyl, isohexyl, 2-methylpentyl and 3-methylpentyl. Lower alkyl groups such as methyl, ethyl, n-propyl, isopropyl, n-butyl, isobutyl, sec-butyl and tert-butyl are preferred.

Examples of the "cycloalkyl group" are those having 3 to 7 carbon atoms, such as cyclopentyl, cyclohexyl and cycloheptyl. Cyclohexyl is preferred.

The "aryl group" may be mononuclear or polynuclear, and the aromatic ring may have at least one, preferably only one, substituent selected from lower alkyl, lower alkoxy and nitro groups. Examples of such substituted or unsubstituted aryl groups include phenyl, p-tolyl, o-tolyl, m-tolyl, p-methoxyphenyl, m-methoxyphenyl, o-methoxyphenyl, p-nitrophenyl, m-nitrophenyl, α-naphthyl, and β-naphthyl. Of these, phenyl, tolyl and nitrophenyl are especially suitable.

The "aralkyl group" includes aryl-substituted lower alkyl groups in which the aryl moiety has the same meaning as given above. Examples are benzyl, phenethyl, p-nitrobenzyl, m-nitrobenzyl, o-nitrobenzyl, o-methoxybenzyl, m-methoxybenzyl, p-methoxybenzyl, p-cyanobenzyl, α-methylbenzyl, α-naphthylmethyl and β-naphthylmethyl. Benzyl and mono-substituted benzyl groups such as p-nitrobenzyl are preferred.

The "4- to 7-membered monovalent heterocyclic group containing 1 to 4 hetero atoms selected from nitrogen, oxygen and sulfur atoms" for $R_3$ may be aliphatic or aromatic and contain only one hetero atom, or two hetero atoms (nitrogen and oxygen atoms, nitrogen and sulfur atoms, or oxygen and sulfur atoms). The heterocyclic group may be substituted by at least one, preferably only one, substituent selected from the class consisting of lower alkyl, lower alkoxy and nitro groups. Specific examples of such heterocyclic groups are given below.

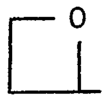 , 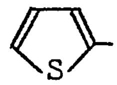 , 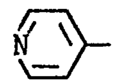 , 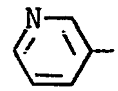

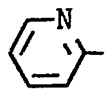 , 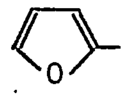 , 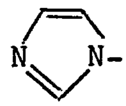 , 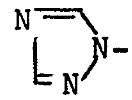

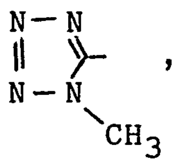 , 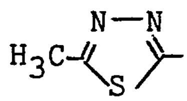 , 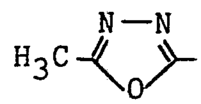 ,

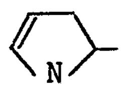 , 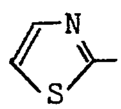 , 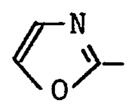 , 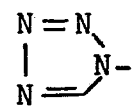

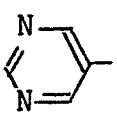 , 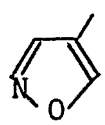 , 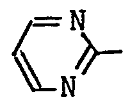 , 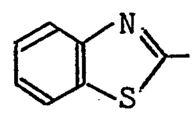 ,

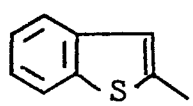 , 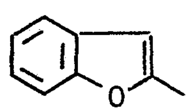 , 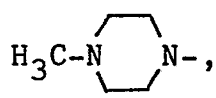 , and 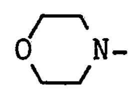 .

Preferred heterocyclic groups include the following:

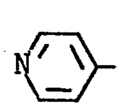 , 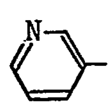 , 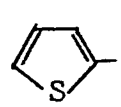 , 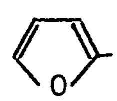 ,

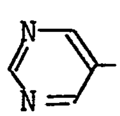 , and 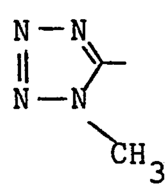 .

In the group $-R_4-Z-R_5$ for $R_3$ in formula (I) or (I-b), examples of the "lower alkylene group which may be substituted by a hydroxyl or lower alkanoyloxy group" are as follows:

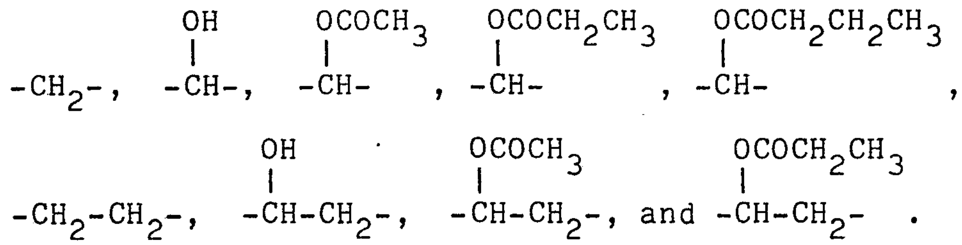

Especially preferred species are as follows:

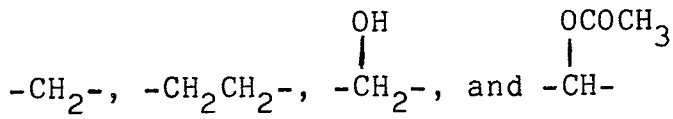

The alkyl group, cycloalkyl group, aryl group, aralkyl group and 4- to 7-membered heterocyclic group for $R_5$ have the aforesaid meanings. Thus, specific exmples of the group $-R_4-Z-R_5$ are as follows:

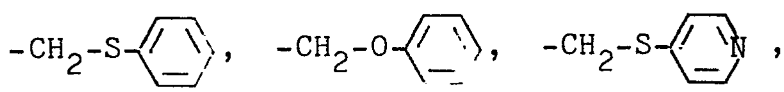

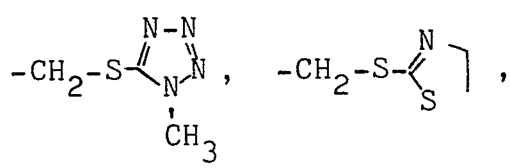

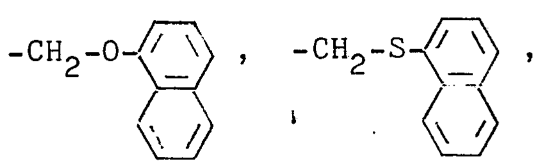

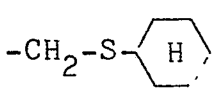 , $-CH_2-S-CH(CH_3)_2$,

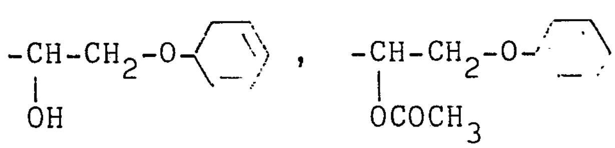

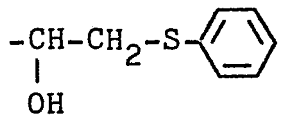 , 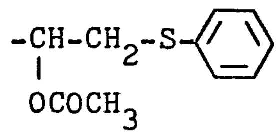 ,

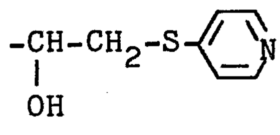 , 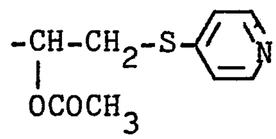 ,

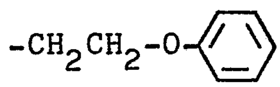 , and 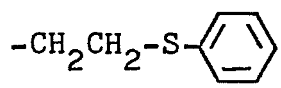 .

A group of especially preferred compounds of formula (I) provided by this invention are those in which A represents a hydrogen atom, and those in which A represents the group $-Y-R_3$ in which Y is -CO- and $R_3$ is a group of the following formula

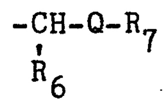

in which $R_6$ represents a hydrogen atom, a hydroxyl group or an acetoxy group, $R_7$ represents a lower alkyl group, a phenyl group which may be substituted by one methyl or nitro group, or a pyridyl group, and Q represents a direct bond or an oxygen or sulfur atom.

Typical examples of the compounds of formula (I) provided by this invention are given below excepting those specifically shown in Examples to be given hereinbelow.

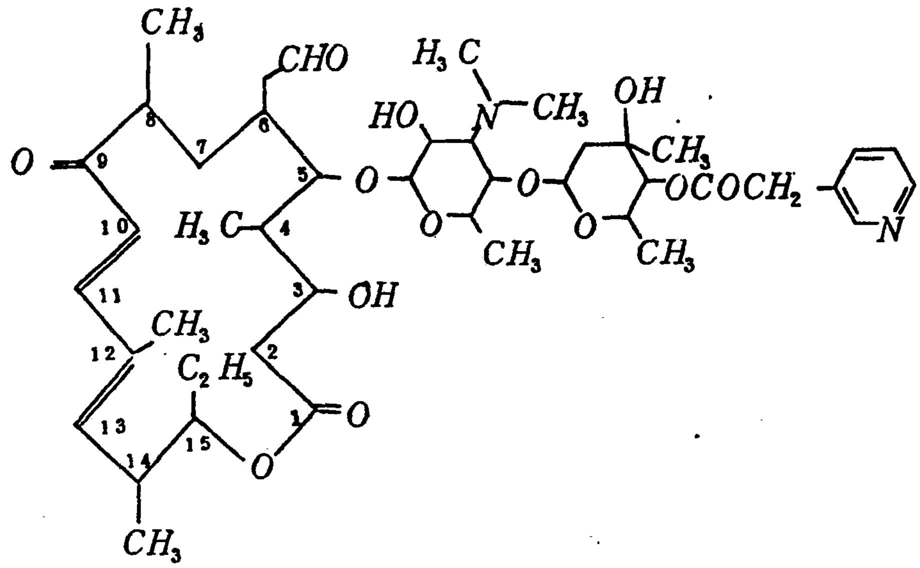

(The macrocyclic lactone moiety will be abbreviated

hereinbelow as follows: 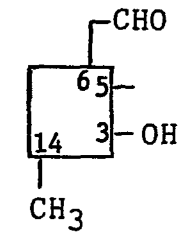 )

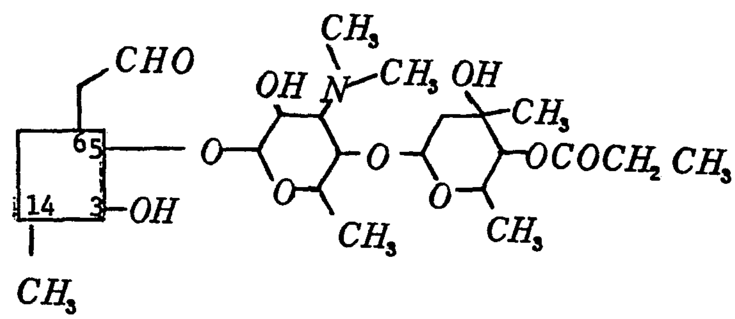 ,

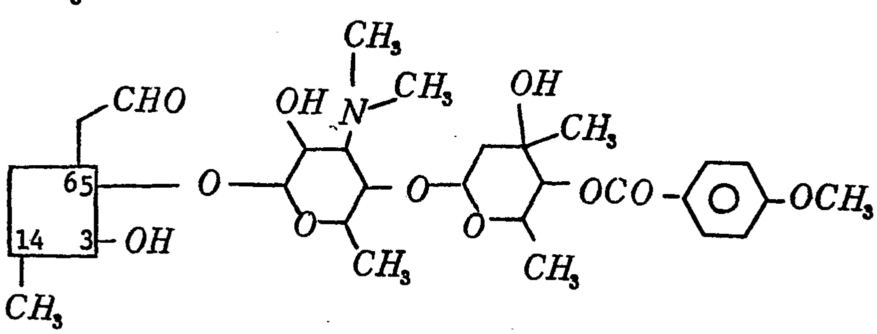 ,

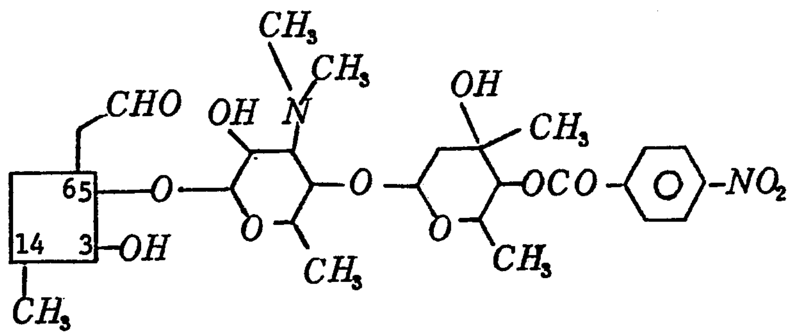

,

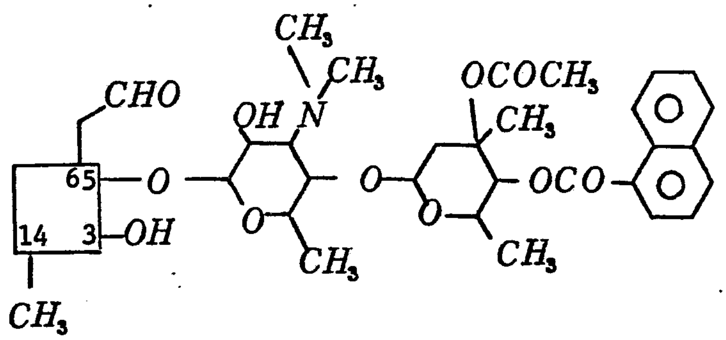

,

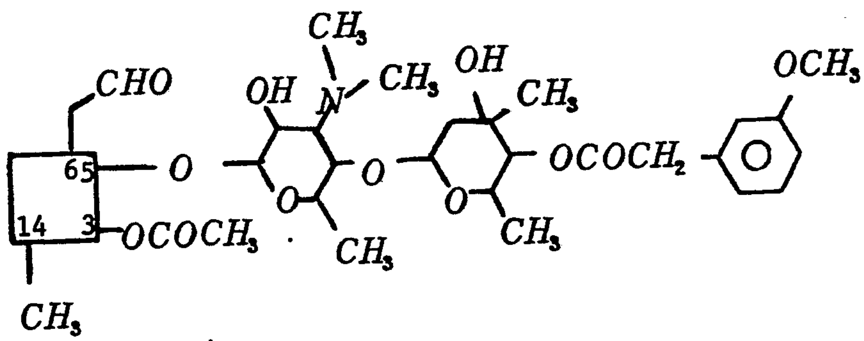

,

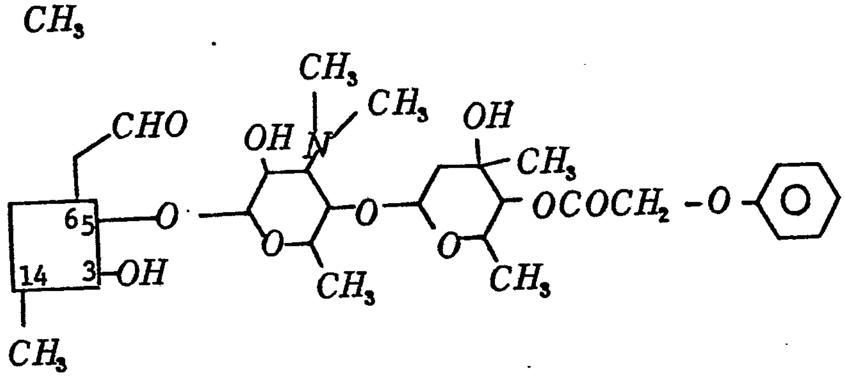

,

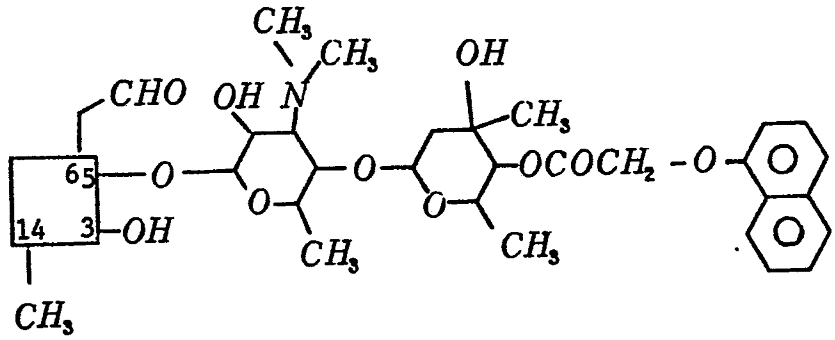

,

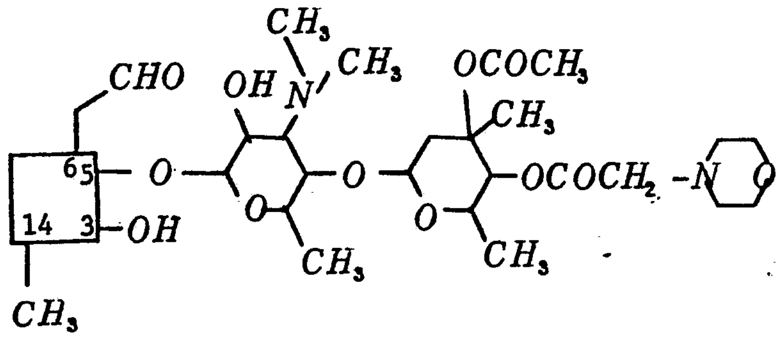
,

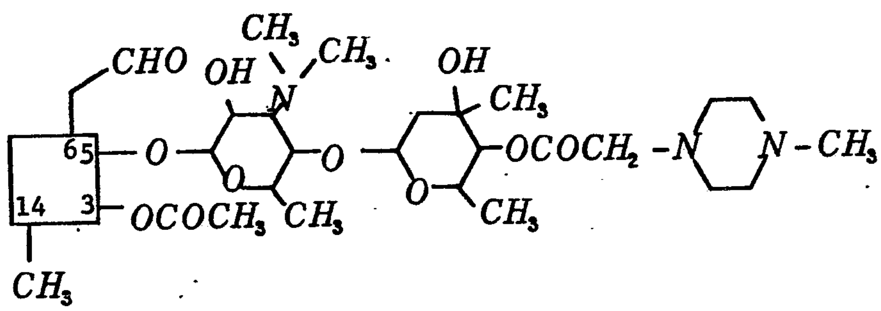
,

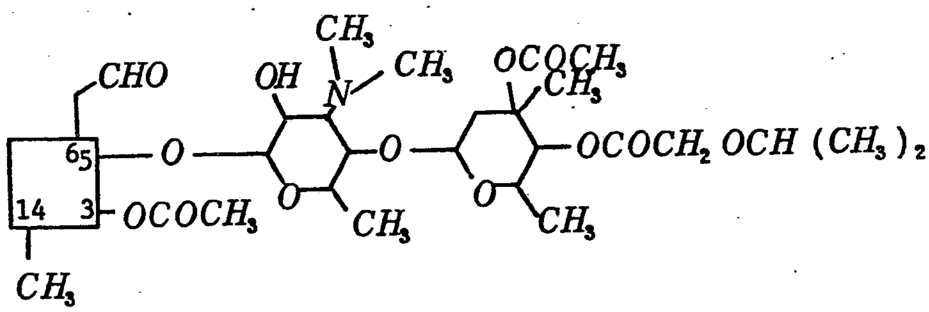
,

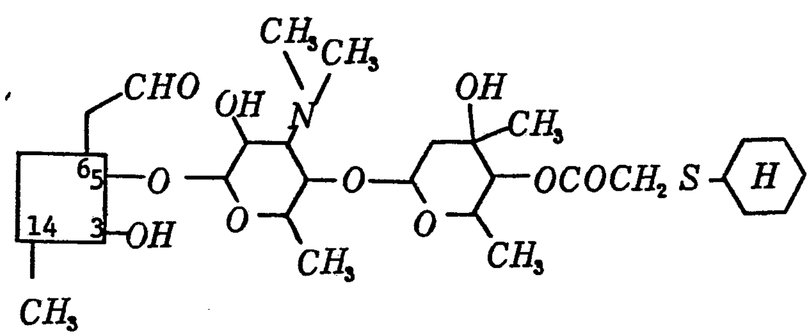
,

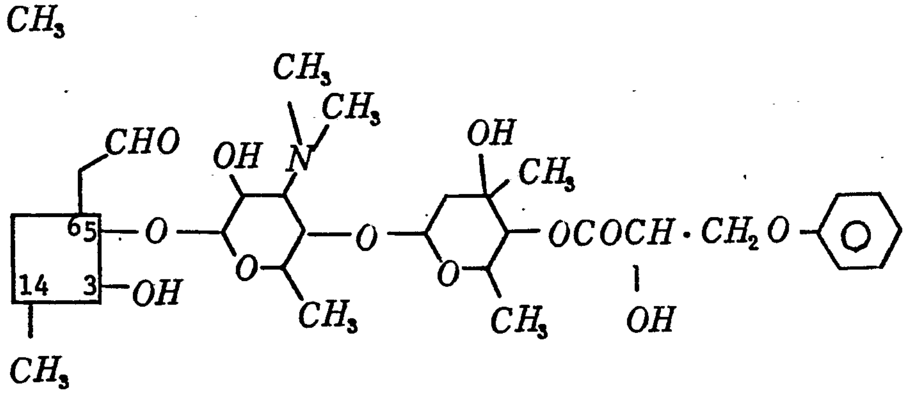
,

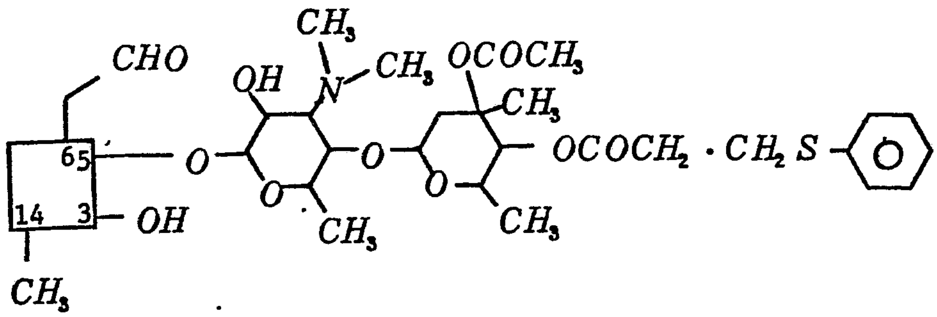 ,

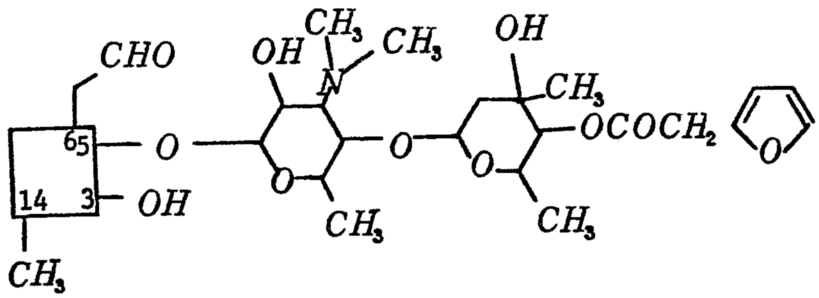 ,

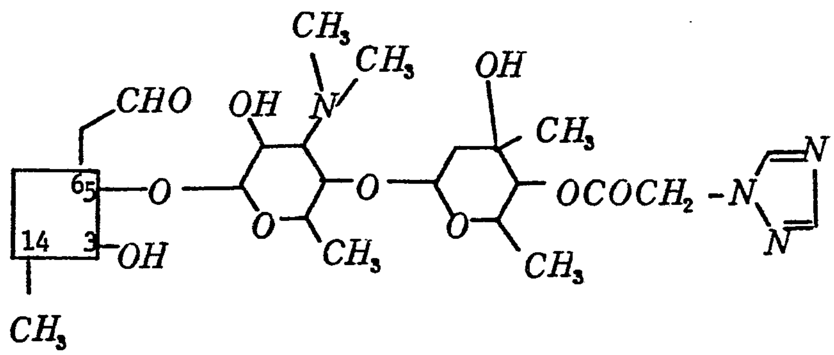 ,

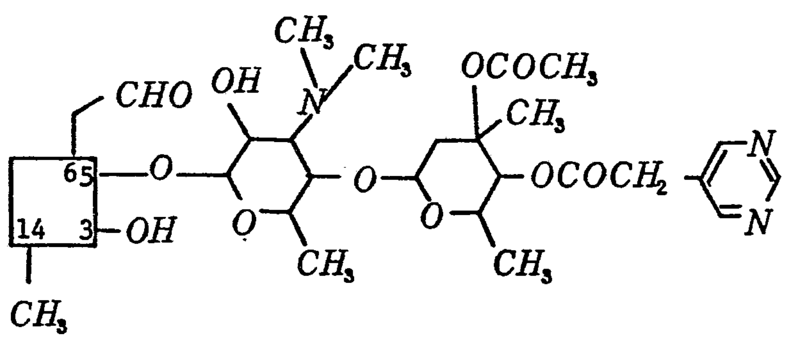 ,

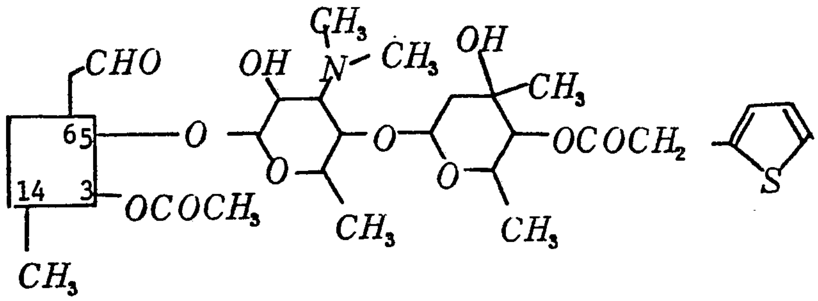

,

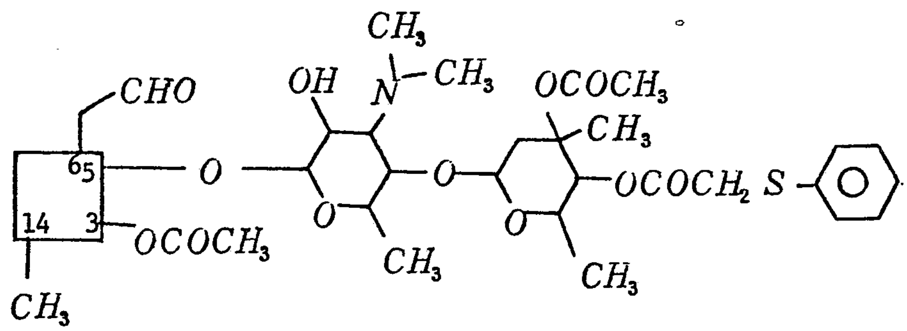

,

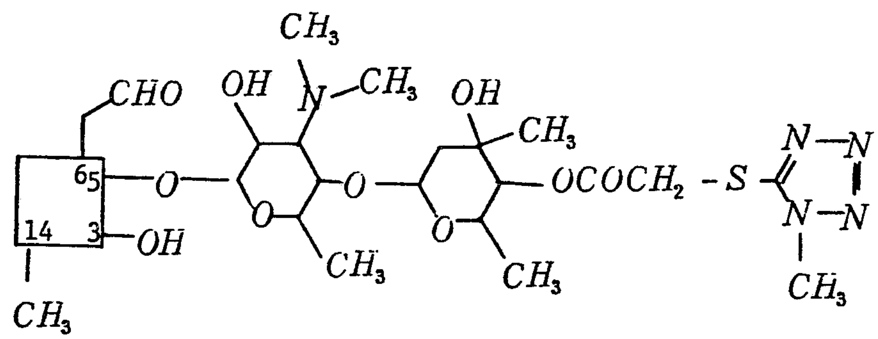

,

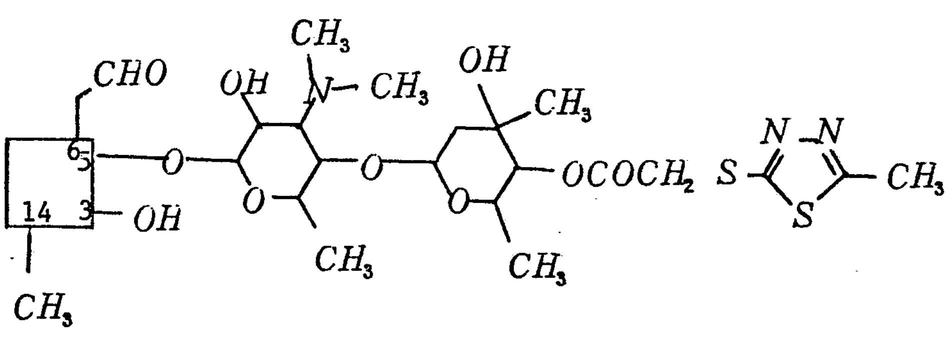

,

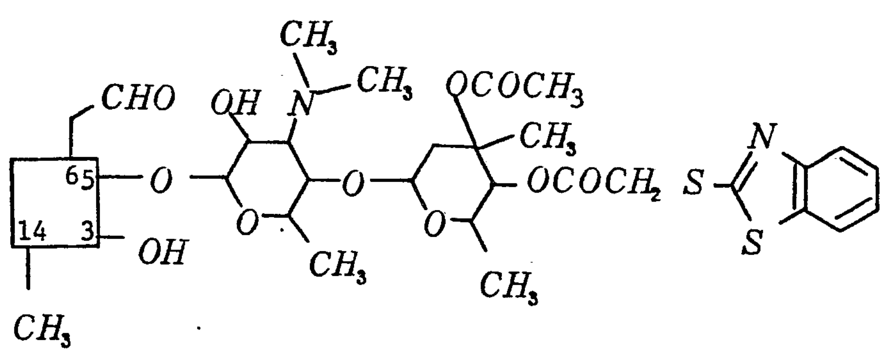

Since the compounds of formula (I) have a basic amino group in the molecule, they may exist in the form of an acid addition salt. Examples of such acid addition salts include addition salts with inorganic acids such as hydrochloric acid, sulfuric acid, and phosphoric acid, and organic acids such as acetic acid, tartaric acid, propionic acid, citric acid and succinic acid. Pharmaceutically acceptable acid addition salts are preferred.

The compounds of formula (I) can be produced by various processes described below depending upon the types of substituents.

(A) The antibiotic YT-3927 of formula (I-a) can be produced by a process which comprises cultivating a microorganism having the ability to produce the antibiotic YT-3927 in a nutrient medium to produce and accumulate the antibiotic YT-3927 in the nutrient medium, and recovering the antibiotic YT-3927 from the culture broth.

The microorganism having the ability to produce the antibiotic YT-3927 used in this process may be those belonging to any genus so long as they have the ability to produce the antibiotic YT-3927 having the physicochemical properties given hereinabove. Generally, microorganisms of the genus Streptomyces which have the ability to produce the antibiotic YT-3927, particularly Streptomyces fradiae YT-3927, which is a new mutant of Streptomyces fradiae NRRL B-2702 having the ability to produce tylosin, can be advantageously used.

The new mutant Streptomyces fradiae YT-3927 can be produced by mutating the parent strain Streptomyces fradiae NRRL B-2702 with N-methyl-N'-nitro-N-nitrosoguanidine as a mutagenic agent in a customary manner. In the present invention, not only the above new mutant, but also other microorganisms such as Streptomyces fradiae NRRL B-2702 to which the ability to produce the antibiotic YT-3927 has been imparted by ordinary microorganism species improving methods, for example mutating means using chemicals other than the aforesaid mutagenic agents,

ultraviolet light, radiation, etc., or gene manipulating means such as conjugation, transformation, transduction and cell fusion can be used in this invention.

The Streptomyces fradiae YT-3927 having the ability to produce the antibiotic YT-3927 has the following microbiological properties.

(1) Morphology

In an inorganic salt starch agar medium (at 30°C for 14 days), the spore chain at the ends of well branched aerial mycelia looks like a hook, loop or irregular coil (Retinaculiariti section). The spores are spherical to elliptical with a size of about (0.7 - 1.0) x (0.9 - 1.5) microns. Many of them are relatively short rods, and a chain of 20 to 50 spores rarely occurs. The structure of the spore surfaces is smooth, but in rare cases, is nodular.

(2) Cultural characteristics

The growth in various culture media is summarized in Table 1. The cultivation was carried out at 30°C for 14 days. The colors were expressed mainly by the method of H. D. Tresner and E. J. Backus: J. Appl. Microbiol., Vol. 11, No. 4 (1963), pages 335 - 338.

Table 1

| Culture medium | Growth | Color of aerial mycelia | Color of substrate mycelia | Soluble pigment |
|---|---|---|---|---|
| Sucrose nitrate agar medium | Moderate | White (a) | Pale yellow (2ca - 2db) | None |
| Glucose asparagine agar medium | Moderate | White to slightly yellowish white | Pale yellow (2ca - 2da) | None |
| Glycerol asparagine agar medium (ISP-5 medium) | Good | White (a) | Pale yellow (2db) to light yellow (2fb) | None |
| Starch agar medium (ISP-4) | Good | White to slightly brownish white | Pale yellow (2db) to light yellow (2fb) | None |
| Tyrosine agar medium (ISP-7 medium) | Good | Yelowish white | Yellow brown | None |
| Nutrient agar medium | Good | White (a) | Pale yellow (2db) to light yellow (2fb) | None |
| Yeast malt agar medium (ISP-2 medium) | Good | White (a) | Light yellow (2fb) to brown yellow | None |
| Oatmeal agar medium (ISP-3 medium) | Good | Yellowish white | Light yellow (2fb) | None |

(3) Physiological properties

a) Growth temperature range:

Grows at 10 to 42°C, and the optimal temperature range is 25 to 37°C.

b) Liquefaction of gelatin (cultivated at 20°C in a glucose peptone gelatin medium):

Hardly liquefied, or only slightly liquefied.

c) Hydrolysis of starch (on a starch agar medium):

Hydrolyzed

d) Coagulation and peptonization of skimmed milk:

Peptonized with coagulation

e) Formation of a melanoid pigment:

No melanoid pigment formed in a tyrosine agar medium and a peptone yeast iron agar medium.

(4) Utilization of various carbon sources (in a Pridham and Gottlieb agar medium):

| | |
|---|---|
| L-arabinose | - to ± |
| D-xylose | + |
| D-glucose | + |
| D-fructose | + |
| Sucrose | + |
| Inositol | + |
| L-rhamnose | - |
| raffinose | - to ± |
| L-mannitol | + |

(+: well utilized; ±: slightly utilized;
-: not easily utilized)

It is seen from the above results that although Streptomyces fradiae YT-3927 has much the same properties as the parent strain, Streptomyces fradiae NRRL B-2702, it is a new mutant strain which is clearly distinguished from the parent strain in that it has the ability to

produce a different antibiotic from the parent strain.

The Streptomyces fradiae YT-3927 is deposited under FERM P-5758, FERM BP-12 in Fermentation Research Institute of Agency of Industrial Science and Technology, Japan at 1-1-3 Higashi, Yatabe-cho, Tsukuba-gun, Ibaraki-ken, Japan.

Cultivation of the above strain capable of producing the antibiotic YT-3927 can be carried out by inoculating it in a nutrient medium, and cultivating it by a known method of cultivating actinomycetes.

Conventional carbon sources, nitrogen sources, inorganic salts, etc. used for cultivation of actinomycetes can be used as nutrient sources to be included in the culture medium. Examples of the carbon sources are glucose, starch, maltose, glycerol, soybean oil, colza oil used either singly or in combination. The nitrogen sources include organic nitrogen sources such as meat extract, fish meal, peptone, dried yeasts, yeast extracts, wheat germ, soybean flour, gluten meal and cottonseed residues; and inorganic nitrogen sources such as ammonium sulfate, sodium nitrate and urea. If desired, inorganic salts such as sodium chloride, calcium carbonate, phosphates and magnesium sulfate may be added. Furthermore, it is possible to add organic or inorganic materials which promote the growth of the microorganism or the production of the antibiotic YT-3927.

The cultivation of the strain capable of producing the antibiotic YT-3927 in such a nutrient medium can be carried out in accordance with conventional methods used in the production of antibiotics by general actinomycetes. Generally, it is preferred to perform the cultivation under aerobic conditions. Usually, it can be performed with stirring and aeration. The method of cultivation may be stationary, shaken or submerged with agitation and aeration. Submerged cultivation, especially submerged cultivation with agitation under aerobic conditions, is preferred.

The cultivation temperature may be any temperature which does not substantially inhibit the growth of the antibiotic YT-3927-producing microorganisms and can give the antibiotic YT-3927. Generally, the suitable cultivation temperature is 20 to 40°C, preferably 25 to 35°C. The pH of the culture medium is generally 4.5 to 8.5, preferably about 6 to about 8.

In the case of large-scale cultivation, it is advantageous to prepare a seed culture, inoculate it in a nutrient medium, and then cultivate it in a submerged condition.

The cultivation can usually be continued until the antibiotic YT-3927 is fully accumulated in the culture medium. The cultivation time varies depending upon the composition of the culture medium, the cultivation temperature, the microorganism strain used, etc. In submerged cultivation, it is 2 to 12 days.

The amount of the antibiotic YT-3927 accumulated in the culture medium can be determined by usual methods, for exmple by bioassay or chromatography using sensitive microorganisms. The optimum amount of the antibiotic accumulated can be easily determined by this method. The antibiotic YT-3927 is recovered and isolated when the amount of accumulation reaches its maximum.

Since the antibiotic YT-3927 is a basic macrolide antibiotic, the recovery and isolation of the antibiotic YT-3927 can be performed by methods normally used for the purification and isolation of basic macrolide antibiotics, for example by an extraction method based on the utilization of the differences in solubility in suitable solvents, and a fractionating method based on the differences in the utilization of adsorptive and affinitive power for adsorbents, etc. Such methods may be used in suitable combinations, or repeated.

One specific examples of recovery of the antibiotic YT-3927 from the culture broth and its purification is shown below.

The culture broth is made weakly alkaline with sodium hydroxide or the like, and extracted with a solvent such as ethyl acetate, butyl acetate, chloroform, benzene or toluene. The solvent layer was separated, and an acidic aqueous solution such as a 1/5 M sodium acetate-HCl buffer, is added. The mixture is transferred to the aqueous layer. The aqueous layer is then made weakly alkaline with sodium hydroxide or the like, and extracted with a solvent such as ethyl acetate, butyl acetate, chloroform, benzene or toluene. The solvent layer is separated, and concentrated to dryness under reduced pressure. The resulting pale yellow white powder is then chromatographed on a silica gel column using a mixed solvent such as benzene-acetone and benzene-methanol as an eluent. Fractions containing the antibiotic YT-3927 are collected, concentrated to dryness under reduced pressure, dissolved in ethyl acetate, butyl acetate, chloroform, benzene, toluene, etc., and transferred into acidified water. Then, the water layer is neutralized with alkali, and the product is extracted with the same solvents as described before. The solvent layer is concentrated to dryness under reduced pressure to give a pure white powder of the antibiotic YT-3927.

The antibiotic YT-3927 in the above process can be assayed by developing it with a 2:1 mixture of acetone and benzene by the ascending method using a silica gel thin-layer chromatographic plate (SILICA GEL 60 $F_{254}$, a product of Merck Co.), and measuring the UV absorption value at 283 nm by a chromatographic scanner (Model CS-910, a product of Shimadzu Seisakusho Co., Ltd.). The antibiotic YT-3927 has an Rf value of 0.19.

[B] Compounds of formula (I-b) in which $R_2$ is a hydrogen atom can be produced by reacting a compound of the following formula

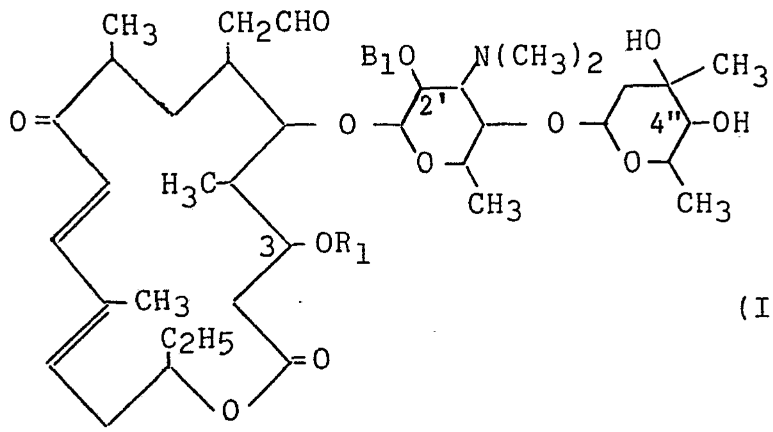

(II)

wherein $B_1$ represents a protective group for a hydroxyl group, and $R_1$ is as defined above,

or its salt, with a reactive derivative of an acid represented by the following formula

$$R_3\text{-Y-OH}$$

wherein $R_3$ is as defined above,

to introduce the group $R_3$-Y- into the hydroxyl group at the 4"-position of the compound of formula (II), and thereafter, splitting off the protective group $B_1$.

Introduction of the $R_3$-Y- group can be carried out by known acylation methods. For example, the reaction is carried out in the absence of a solvent or in the presence of a suitable inert solvent such as methylene chloride, chloroform, acetone, benzene, toluene, tetrahydrofuran or acetonitrile. The reaction can be carried out generally at about -30°C to the refluxing temperature of the reaction mixture, preferably at about -20°C to 60°C. However, when the starting material is a compound of formula (II) in which $R_1$ is hydrogen and the acylation of the hydroxyl group at the 3-position is not desired, it is desirable to use relatively mild acylating conditions, i.e. temperatures of approximately up to room temperature.

Examples of the reactive derivatives of the acid of formula (III) used as the acylating agent in the above

reaction are acid halides (particularly, chlorides), acid anhydrides, and mixed acid anhydrides (e.g., anhydrides of the acid of formula (III) and pivalic acid). The amount of the reactive derivative of the acid (III) is not strictly restricted. Generally, it is 1 to 50 moles, preferably 2 to 20 moles, per mole of the compound of formula (II).

When the reactive derivative of a lower alkanoic acid is used as the acylating agent to acylate a compound of formula (II) in which $R_1$ is a hydrogen atom, it is possible to produce a compound of formula (I-b) in which $R_1$ and $R_3$-Y- are lower alkanoyl groups by simultaneously acylating the hydroxyl group at the 4"-position and the hydroxyl group at the 3-position. Conveniently, this simultaneous acylation is carried out at a relatively high temperature of about 20 to about 30°C by using the acylating agent in an amount of 5 to 50 moles, preferably 10 to 20 moles, per mole of the compound of formula (II).

As required, the acylating reaction may be carried out in the presence of an acid binder. Examples of acid binders are organic bases such as pyridine, collidine, N-methylpiperidine, triethylamine and dimethylaniline. The acid binder may be used in an amount of 2 to 50 equivalents, preferably 2 to 30 equivalents, per mole of the compound of formula (II). A liquid organic base such as pyridine may be used concurrently as a solvent by using it in excess.

Removal of the protective group $B_1$ from the acylated product can be effected by a method known *per se*. For example, it may be carried out by dissolving the product in a lower alcohol such as methanol or ethanol, or a hydrous organic solvent, or adding ammonia or an alkali to a hydrous organic solvent having the aforesaid product dissolved or dispersed therein, and treating it at a temperature ranging from room temperature to the boiling point of the solvent for 5 hours to 24 hours.

The resulting compound of formula (I-b) in which

$R_2$ is hydrogen can be separated from the reaction mixture and/or purified by a method known *per se*, for example by pouring the reaction mixture into a solvent such as benzene, toluene, or ethyl acetate, washing it with a saturated aqueous solution of sodium hydrogen carbonate in a customary manner, and removing the solvent under reduced pressure, and if further required, subjecting it to column chromatography.

The compounds of formula (II) used as a starting material in the aforesaid acylating reaction are novel compounds not described in the prior literature. Compounds of formula (II) in which $R_1$ is a hydrogen atom can be produced by introducing a protective group into the hydroxyl group at the 2'-position of the antibiotic YT-3927 of formula (I-a) or its salt by a method known *per se*. The protective group $B_1$ for the hydroxyl group may be a group which can be easily split off by hydrolysis. Examples include acyl groups such as acetyl, monochloroacetyl, trichloroacetyl and propionyl, and acyloxy groups such as butyloxycarbonyl, tert-butyloxycarbonyl or benzyloxycarbonyl.

(C) Compounds of formula (II) in which $R_1$ is a lower alkanoyl group can be produced by acylating the antibiotic YT-3927 of formula (I-a) by an enzymatic method to introduce a lower alkanoyl group selectively into the 3-position, and then introducing a hydroxyl protecting group into the 2'-position. The enzymatic method used for selective acylation of the antibiotic YT-3927 is known *per se*, and is disclosed, for example, in J. Ferment. Technol., Vol. 57, No. 6, pages 519 - 528 (1979).

(D) Compounds of formula (I-b) in which $R_2$ is a lower alkanoyl can be produced by reacting a compound of the following formula

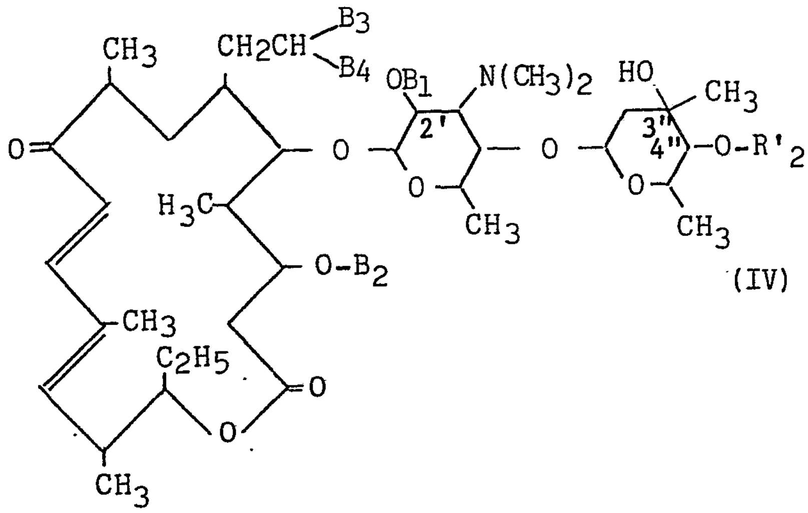

(IV)

wherein $B_1$ is as defined above; $R'_2$ represents a lower alkanoyl group; $B_2$ represents a lower alkanoyl group; and $B_3$ and $B_4$ represent an acetal residue, or when $B_3$ represents a lower alkanoyloxy group, $B_2$ and $B_4$ may together form a direct bond, with an anhydride or mixed anhydride (for example, a mixed acid anhydride of the following acid and pivalic acid) of an acid of the following formula

$$R_3 - Y - OH \qquad (III)$$

wherein $R_3$ and Y are as defined above, in benzene in the presence of 4-dimethylaminopyridine under reflux (at a temperature of about 70 to about 90°C) to perform transfer of the lower alkanoyl group ($R_2$) at the 4"-position to the 3"-position and the introduction of the group $R_3$-Y- into the 4"-position, and thereafter removing the protective group.

The amount of 4-dimethylaminopyridine used as a catalyst in the above raction is generally 0.1 to 10 moles, preferably 1 to 2 moles, per mole of the compound of formula (IV). The anhydride or mixed anhydride of an acid of formula (III) as an acylating agent for the 4"-position is conveniently used in an amount of generally 10 to 50 moles, preferably 30 to 40 moles, per mole of the compound of formula (IV).

As a result of the above reaction, the transfer of the lower alkanoyl group ($R_2$) at the 4"-position to the hydroxyl group at the 3"-position and the introduction of the group $R_3$-Y- into the 4"-position take place to give compounds of the following formula

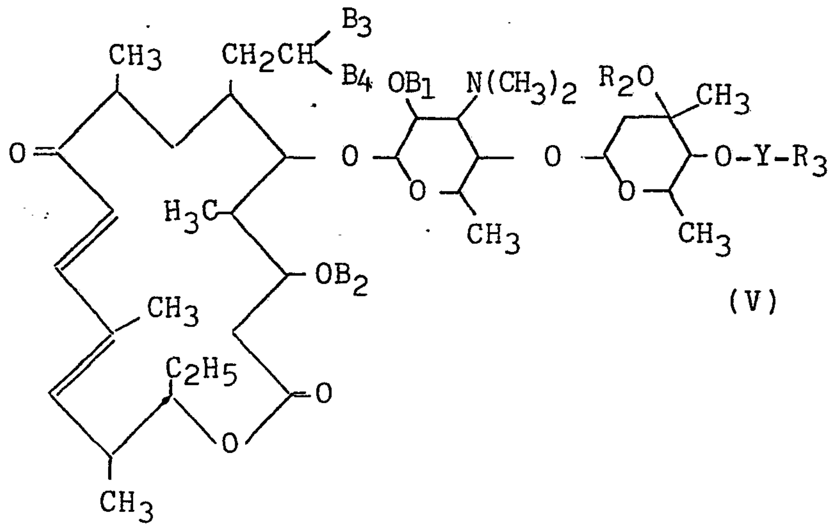

(V)

wherein $R_2$, $R_3$, $B_1$, $B_2$, $B_3$ and $B_4$ are as defined hereinabove.

Subsequent selective hydrolysis of the compound of formula (V) can give compounds of formula (I-b) in which $R_2$ is a lower alkanoyl group.

The compound of formula (IV) used as a starting material in the aforesaid method can be produced by acetalizing the aldehyde at the 20 position of a compound of the following formula

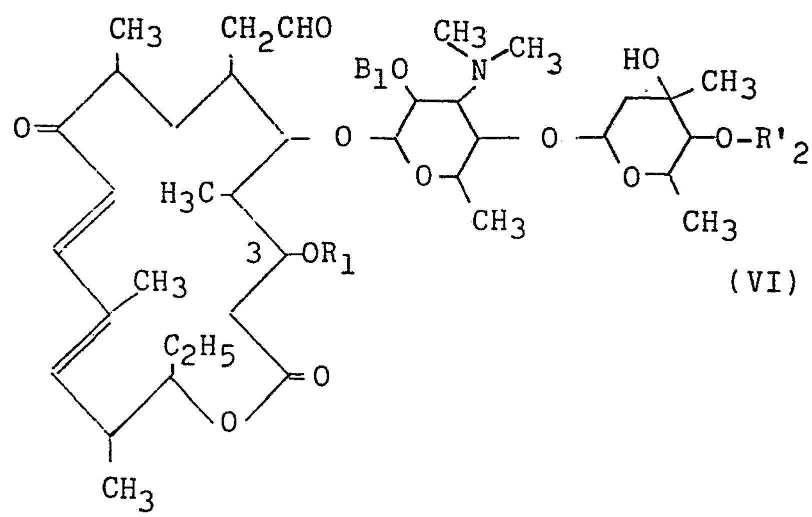

(VI)

wherein $R_1$, $R'_2$ and $B_1$ are as defined above, obtained by the aforesaid acylating reaction, or when $R_1$ is a hydrogen atom, reacting such a compound with a lower alkanoic acid anhydride in the presence of an alkali carbonate.

The compounds of formula (I) prepared by the above processes can be converted by methods known per se into acid addition salts by treatment with hydrochloric acid, phosphoric acid, sulfuric acid, tartaric acid, succinic acid, propionic acid, citric acid, and so on.

The compounds of formula (I) and their salts provided by this invention have excellent antibacterial activity against various pathogenic microorganisms such as Gram-positive bacteria, Gram-negative bacteria and mycoplasmas, and exhibit high bioavailability. Their high activity is demonstrated by the following tests.

(1) Antimicrobial activity

The minimum growth inhibitory concentrations (MIC) of the compounds (I) against various microorganisms were measured by a tube dilution method in a brain heart infusion broth (pH 7.5) as a medium. The results are shown in the following table.

Table 2-1: Antimicrobial spectrum of the antibiotic YT-3927

| Test organisms | Minimum growth inhibitory concentration (μg/ml) | |
|---|---|---|
| | Antibiotic YT-3927 | Tylosin |
| *Staphylococcus aureus* MS10225 (*) | 0.78 | 3.12 |
| *Staphylococcus aureus* 209P | 0.39 | 0.78 |
| *Sartina lutea* PCI 1001 | <0.2 | <0.2 |
| *Bacillus subtilis* NRRL B-558 | 0.39 | 0.78 |
| *Corynebacterium bovis* 1810 | 0.39 | <0.2 |
| *Escherichia coli* NIHJ | 25 | 100 |
| *Klebsiella pneumoniae* PCI602 | 3.12 | 25 |
| *Shigella dysenteriae* JS 11910 | 1.56 | 25 |
| *Mycoplasma gallisepticum* KP-13 | 0.02 | 0.02 |

(*): resistant to tylosin

Table 2-2: Antimicrobial activity (MIC, μg/ml)

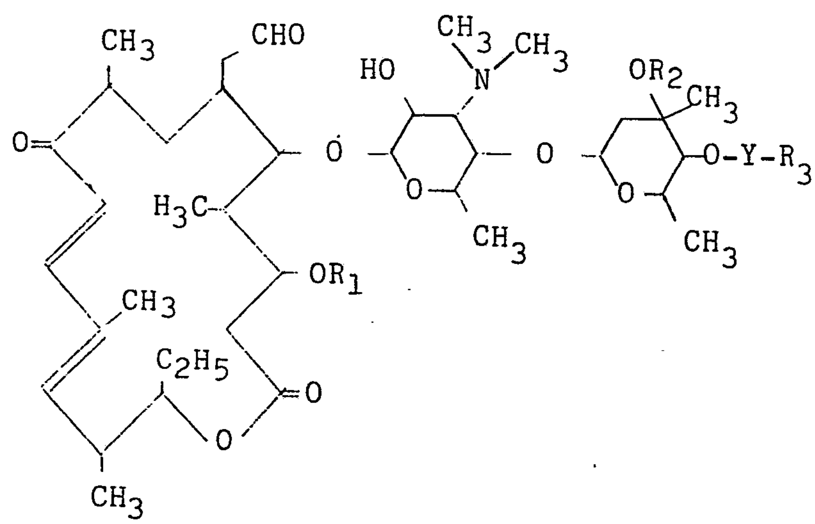

| $R_1$ | $R_2$ | $YR_3$ | Staphylococcus aureus 209P | Staphylococcus aureus MS9861 | Bacillus subtilis NRRL B558 | Klebsiella pneumonae PCI602 |
|---|---|---|---|---|---|---|
| H | H | $COCH_2CH(CH_3)_2$ | 0.4 | 1.6 | 0.4 | 6.3 |
| H | $COCH_3$ | $COCH_2CH(CH_3)_2$ | 0.8 | 1.6 | 0.8 | 25 |
| $COCH_3$ | H | $COCH_2CH(CH_3)_2$ | 0.4 | 6.3 | 0.8 | 25 |
| $COCH_3$ | $COCH_3$ | $COCH_3$ | 1.6 | 3.1 | 0.4 | 100 |
| H | H | $COCH_2$- 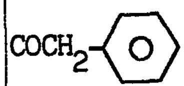 | 0.2 | 3.1 | 0.4 | 3.1 |
| H | H | $COCH_2S$- 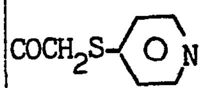 | <0.2 | 1.6 | <0.2 | 3.1 |
| H | H | $COCH_2$- 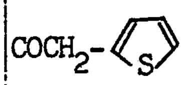 | 0.2 | 1.6 | 0.4 | 3.1 |
| H | $COCH_3$ | $COCH_2S$- 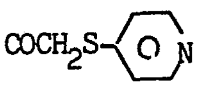 | <0.2 | 0.8 | <0.2 | 6.3 |
| H | H | $COCH_2$- 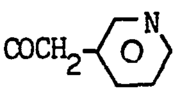 | <0.2 | 0.8 | <0.2 | 1.6 |
| H | $COCH_3$ | $COCH_2$- 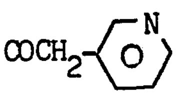 | 0.4 | 1.6 | <0.2 | 3.1 |
| H | H | COCH- 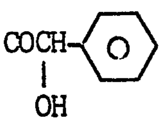 | <0.2 | 1.6 | 1.6 | 3.1 |
| H | H | COCH- 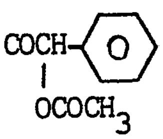 | 0.8 | 3.1 | 0.8 | 25 |
| H | H | CO- 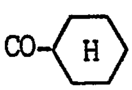 | 0.8 | 3.1 | 0.2 | 12.5 |
| H | $COCH_3$ | CO- 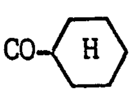 | 1.6 | 6.3 | 0.4 | >100 |
| H | H | CONH- 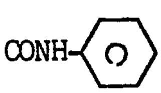 | 0.8 | 3.1 | 0.8 | 6.3 |
| H | H | $-SO_2$- 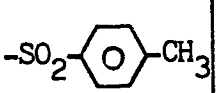 $-CH_3$ | <0.2 | 6.3 | 0.4 | 3.1 |

-to be continued-

Table 2-2: (continued)

| $R_1$ | $R_2$ | $YR_3$ | Staphylococcus aureus 209P | Staphylococcus aureus MS9861 | Bacillus subtilis NRRL B558 | Klebsiella pneumonae PCI602 |
|---|---|---|---|---|---|---|
| H | H | 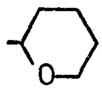 | 1.6 | 3.1 | 0.4 | 3.1 |
| H | H | H | 0.4 | 3.1 | 0.4 | 3.1 |
| Tylosin | | | 0.8 | 6.3 | 0.8 | 25 |

(2) Concentration in the blood

(2-1) The concentration of the antibiotic YT-3927 of formula (I-a) in the blood was measured by bioassay with Sartina lutea using mice. A 0.5% carboxy methyl cellulose solution containing 64 mg/ml of the antibiotic YT-3927 was orally administered to mice in an amount of 0.25 ml per mouse. Then, periodically, the concentration of the antibiotic YT-3927 in the blood was measured. The peak of the blood concentration was noted 1.5 to 2 hours after the administration, and at the peak, the concentration was 10 to 20 μm/ml. In contrast, in the same experiment with tylosin, the peak was noted 1.5 to 2 hours later, but the concentration was less than one-fifth of that of the antibiotic YT-3927.

(2-2) The concentration of each of the antibiotic YT-3927 derivatives of formula (I-b) in the blood was measured by bioassay with Sartina lutea using mice. A 0.5% carboxy methyl cellulose solution containing 16 mg/ml of the antibiotic YT-3927 derivative of formula (I-b) was orally administered to mice in an amount of 0.25 ml per mouse. The concentration of the derivative (I-b) in the blood was periodically measured. The concentration of each of the compounds (I-b) 2 hours after the administration is shown in Table 3.

Table 3: Concentration in the blood (mice)

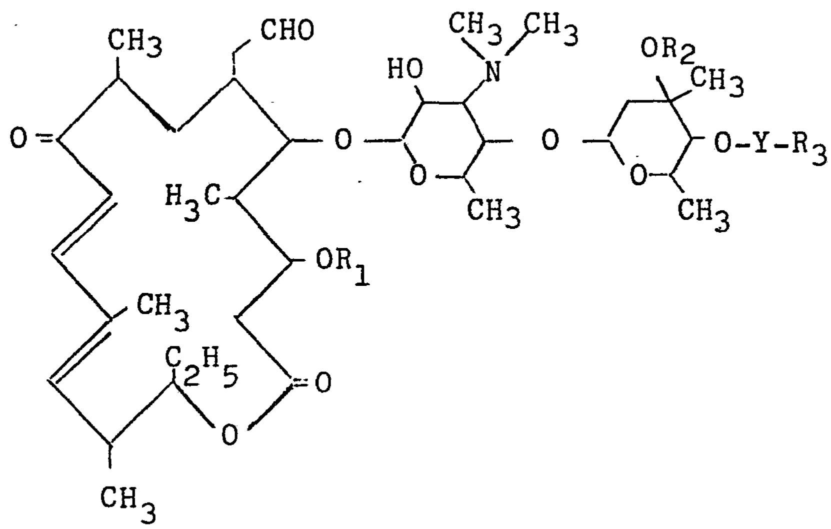

| $R_1$ | $R_2$ | $YR_3$ | Concentration in the blood (mcg/ml) |
|---|---|---|---|
| H | H | $COCH_2CH(CH_3)_2$ | 3.3 |
| H | $COCH_3$ | $COCH_2CH(CH_3)_2$ | 3.1 |
| H | H | $COCH_2$-S- 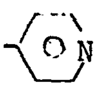 | 4.5 |
| H | $COCH_3$ | $COCH_2$-S- 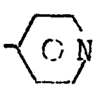 | 3.3 |
| H | $COCH_3$ | $COCH_2$- 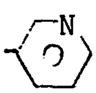 | 3.4 |
| H | H | $SO_2$- 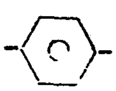 $-CH_3$ | 2.4 |
| H | H | 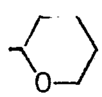 | 2.8 |
| Tylosin | | | 0.7 |

(3) Toxicity

The acute toxicity of the compounds of formula (I) was tested using mice. Each of the compounds was orally administered to mice in an amount of 1000 mg per kg of mouse, and the mice were observed for 2 weeks. In all cases, no toxicity was noted.

The compounds of formula (I) provided by this invention are novel compounds and have excellent antimicrobial activity. Accordingly, they can be used not only as an active ingredient of antibacterial agents for the prevention, therapy and treatment of various bacterial infections, but also for the prevention, therapy and treatment of bacterial infections of animals other than man, for example other mammals, poultry and fish.

For use as a drug for the prevention, therapy and treatment of infections, the compound of formula (I) or its pharmaceutically acceptable salt can be formulated into dosage forms such as capsules, tablets, powders, syrups, ointments, suppositories and injectable preparations by using water, physiological saline, alcohols, vegetable oils, polyethylene glycol, gelatin, lactose, amylose, magnesium stearate, talc, silicic acid, paraffins, aromatic oils, fatty acid monoglycerides or diglycerides, hydroxymethyl cellulose, etc.

The pharmaceutical composition in accordance with this invention may contain the active compound of formula (I) in an amount of about 5 to about 50% by weight, preferably about 10 to about 30% by weight.

The dosage of the compound of formula (I) or its salt can be varied over a wide range of antimicrobially effective amounts depending upon the type of the subject to which it is administered, the severity of the disease of the subject, the judgement of a physician, etc. Generally, it is about 1 to about 100 mg/kg of body weight, preferably about 2 to about 50 mg/kg of body weight, per day.

Furthermore, the compound of formula (I) or its salt can be used as a feed additive for the prevention of infections or the promotion of growth either directly or as a premix.

The compounds of formula (I) are also useful as intermediates for synthesis of new pharmaceuticals.

The following Examples further illustrate the production of the compounds of this invention.

Example 1

(1) Cultivation of Streptomyces fradiae YT-3927

One loopful of a slant culture in which Streptomyces fradiae YT-3927 (FERM P-5758, FERM BP-12) was growing was inoculated in a 500 ml Erlenmeyer flask containing 50 ml of a seed culture medium consisting of 5% glycerol, 1% polypeptone, 0.1 meat extract, 0.1% dipotassium phosphate and 0.1% magnesium sulfate, and cultivated at 30°C for 2 days on a rotating-shaking fermentor. The seed culture broth so obtained was inoculated in a 20-liter jar fermentor containing 10 liters of a culture medium composed of 6% soluble starch, 2% of dry yeast, 0.1% yeast extract, 0.1% dipotassium phosphate, 0.1% magnesium sulfate, 0.5% sodium chloride and 0.4% calcium carbonate, and cultivated at 30°C for 5 days at a rotating speed of 350 rpm while passing air through the fermentor at a rate of 10 liters/min.

(2) Recovery of the antibiotic YT-3927

The culture broth obtained in (1) above was adjusted to pH 8.5 with sodium hydroxide, and then 10 liters of ethyl acetate was added. The mixture was stirred to perform extraction. The ethyl acetate layer was recovered by centrifugation. The resulting ethyl acetate solution was cooled to 5°C, and 5 liters of M/5 sodium acetate-HCl buffer (pH 3.0) was added. The mixture was stirred, and the buffer layer was separated by centrifugal separation. The pH of the buffer layer was adjusted to 8.5 with sodium hydroxide, and 2.5 liters of ethyl acetate was added. The mixture was stirred to perform extraction. The ethyl acetate layer was separated by centrifugation, and concentrated to dryness under reduced pressure. The dried product was washed with a small amount of ether, and dried to give 2.2 g of a pale yellowish white powder.

(3) Purification of the antibiotic YT-3927

The powder obtained in (2) was suspended in a small amount of benzene, and charged onto a column containing

100 g of silica gel (Wakogel C-200) saturated with benzene. The column was eluted with a 2:3 mixture of acetone and benzene as an eluent. The eluate was collected as 10 ml fractions. The fractions which contained the antibiotic YT-3927 were collected and concentrated to dryness. The dryed product was dissolved in 100 ml of ethyl acetate, and then transferred at a low temperature to 100 ml of a 1/5M sodium acetate-HCl buffer (pH 3). The buffer layer was separated and adjusted to pH 8.5 with sodium hydroxide, and again 50 ml of ethyl acetate was added to perform extraction. The ethyl acetate layer was separated, dehydrated, and concentrated to dryness under reduced pressure. The dried product was washed with a small amount of ether and dried to give 1.2 g of a white powder as a purified product. The physicochemical properties of the resulting white powder corresponded exactly with those given hereinabove.

Example 2

2'-Acetyl YT-3927

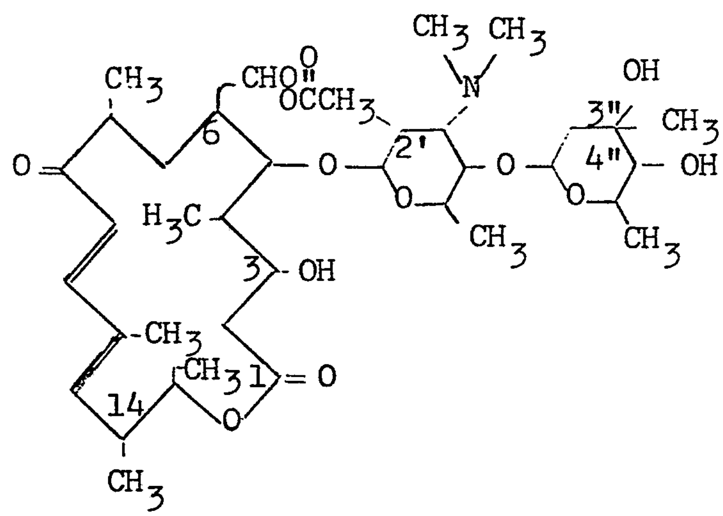

(Hereinafter, the macrocyclic lactone moiety will be abbreviated as follows except in special cases.

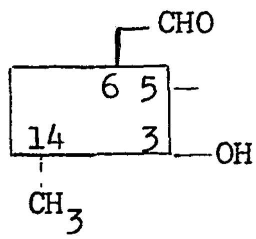

One gram of the antibiotic YT-3927 (to be referred to hereinafter simply as "YT-3927") was dissolved in 5 ml of acetic anhydride, and the solution was stirred at room temperature for 30 minutes. The reaction mixture was poured into 50 ml of water to decompose the excessive acetic anhydride completely. The reaction mixture was then neutralized, and the mixture was extracted with 50 ml of chloroform twice. The combined extracts were washed with a saturated aqueous solution of sodium bicarbonate twice and then with a saturated aqueous solution of sodium chloride once, and dried over anhydrous sodium sulfate. The solution was filtered, and the solvent was removed under reduced pressure, followed by drying. The resulting yellowish white solid was crystallized from a mixture of toluene and ether to give 640 mg of the title compound as white needles.

The physicochemical properties of the product were as follows:

Melting point: 127 -137°C

UV: $\lambda_{max}^{CH_3OH_2}$ 283 nm, $E_{1\,cm}^{1\%}$ = 273

IR (KBr tablet; $cm^{-1}$): 3500, 2970, 2930, 2730, 1750, 1730, 1680, 1595, 1240

NMR (90 MHz, $CD_3Cl$, internal reference TMS):

$\delta_{ppm}$: 1.75 (s, 12-$CH_3$), 2.02 (s, 2'-$OCOCH_3$), 2.34 (s, $N(CH_3)_2$), 4.18 (d, $J_{1',2'}$=7.5Hz, $H_1$), 4.65 (m, $H_{15}$), 4.96 (broad d, $J_{1'',2''ax}$= 3Hz, $H_1$), 5.59 (d, $J_{13,14}$=10.5Hz, $H_{13}$), 6.21 (d, $J_{10,11}$=16Hz, $H_{10}$), 7.23 (d, $J_{10,11}$= 16Hz, $H_{11}$), 9.60 (s, CHO).

Example 3

20,2',4"-triacetyl-3,20-O-cyclo YT-3927

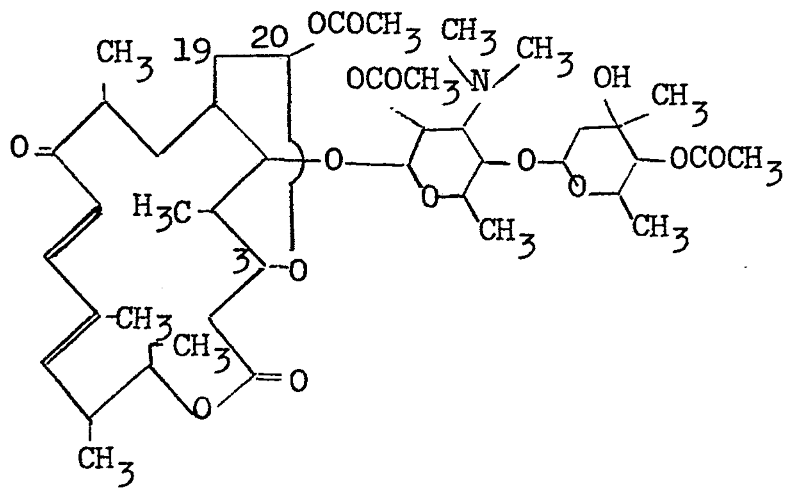

Five grams of YT-3927 was dissolved in 15 ml of acetic anhydride, and 5 g of potassium carbonate was added. The mixture was stirred at 60°C. Twenty-three hours later, 100 ml of water was added to the reaction mixture, and the pH of the mixture was adjusted to 10. The mixture was then extracted with 75 ml of chloroform twice. The combined extracts were washed with water, dried over magnesium sulfate and filtered. The chloroform was removed under reduced pressure to give 5.5 g of a crude powder. The crude powder was purified by silica gel column chromatography (Ø 2.7 cm x 50 cm, benzene-acetone=8:1) to give 3.6 g of the title compound having the following physicochemical properties.

I.R (KBr tablet; $cm^{-1}$):

3470, 2970, 2930, 1740, 1660, 1625, 1575, 1240

NMR (same as in Example 2), δ ppm:

1.94 (s, 12-$CH_3$), 2.03 (s, 20-$OCOCH_3$), 2.08 (s, 2'-$OCOCH_3$), 2.14 (s, 4"-$OCOCH_3$), 2.40 (s, $N(CH_3)_2$), 4.18 (d, $J_{1',2'}$=7.5Hz, $H_{1'}$), 4.59 (d, $J_{4'',5''}$=10Hz, $H_{4''}$), 5.03 (broad, d, $J_{1'',2''}$ ax=3Hz, $H_{1''}$), 5.79 (broad d, $J_{2a,3}$=10Hz, $H_3$), 6.00 (d, $J_{10,11}$=10.5Hz, $H_{13}$), 6.24 (d, $J_{10,11}$=16Hz, $H_{10}$), 6.84 (d, $J_{10,11}$=16Hz, $H_{11}$).

Example 4

3,2',4"-triacetyl YT-3927

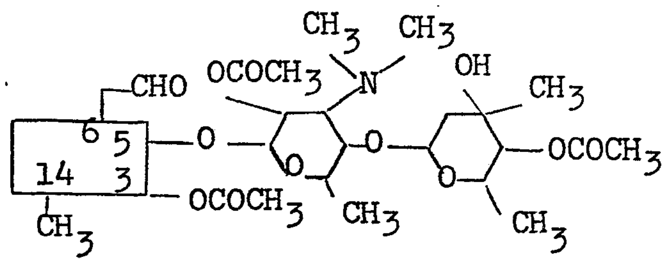

1.0 g of YT-3927 was dissolved in 3 ml of pyridine, and 3 ml of acetic anhydride was added. The mixture was stirred at 20°C for 2 days. The reaction mixture was poured into 50 ml of ice-water to decompose the excessive acetic anhydride completely. Then, the pH of the mixture was adjusted to 7, and it was extracted with 100 ml of toluene. The toluene layer was washed with 50 ml of a saturated aqueous solution of sodium bicarbonate twice and then with 50 ml of a saturated aqueous solution of sodium chloride, and then dried over anhydrous sodium sulfate. The dried solution was filtered, and the solvent was removed under reduced pressure, followed by drying to give 0.8 g of 3,2',4"-triacetyl YT-3927. The product can be used in a subsequent reaction without further purification.

The dried product had the following physicochemical properties.

I.R (KBr tablet, $cm^{-1}$)

3500, 2970, 2930, 2730, 1740, 1680, 1595, 1230

NMR (Same as in Example 1) $\delta_{ppm}$

1.80 (s, 12-$CH_3$), 2.05 (s, 2'-$OCOCH_3$), 2.08 (s, 3-$OCOCH_3$), 2.13 (s, 4"-$OCOCH_3$), 2.40 (s, $N(CH_3)_2$), 4.18 (d, $J_{1',2'}$=7.5Hz, $H_{1'}$) 4.56 (d, $J_{4'',5''}$=10Hz, $H_4$), 5.03 (broad, d, $J_{1'',2''}$ ax=3Hz, $H_{1''}$), 5.12 (broad d, $J_{2a,3}$= 10Hz, $H_3$), 5.74 (d, $J_{13,14}$=10.5Hz, $H_{13}$), 6.23 (d, $J_{10,11}$=16Hz, $H_{10}$), 7.35 (d, $J_{10,11}$= 16Hz, $H_{11}$), 9.62 (s, CHO).

Example 5

3,2',4"-triacetyl YT-3927 dimethyl acetal

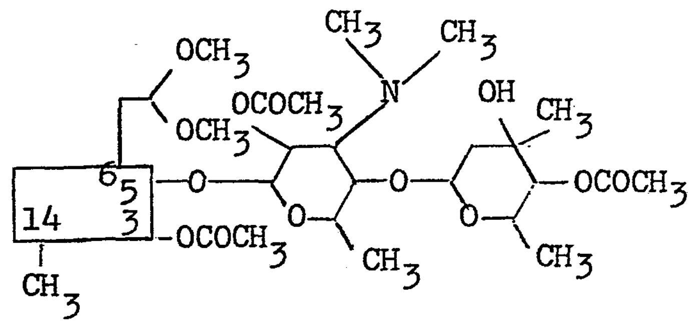

500 mg of 3,2',4"-triacetyl YT-3927 (see Example 4) was dissolved in 25.3 ml of 0.25% hydrochloric acid-methanol, and the solution was stirred at room temperature for 1 hour. The reaction mixture was poured into 100 ml of toluene, washed with 50 ml of a saturated aqueous solution of sodium bicarbonate and 50 ml of a saturated aqueous solution of sodium chloride, and dried over anhydrous sodium sulfate. The dried solution was filtered, and the solvent was removed under reduced pressure, followed by drying to give 524 mg of 3,2',4"-triacetyl YT-3927 dimethyl acetal. This product can be used in a subsequent reaction without further purification.

The dried product had the following physicochemical properties.

I.R (KBr tablet, $cm^{-1}$):

3500, 2970, 2930, 1740, 1680, 1595, 1230

NMR (Same as in Example 2) $\delta_{ppm}$:

1.78 (s, 12-$CH_3$), 2.06 (s, 2'-$OCOCH_3$), 2.09 (s, 3-$OCOCH_3$), 2.13 (s, 4"-$OCOCH_3$), 2.40 (s, $N(CH_3)_2$), 3.20 (s, $OCH_3$), 3.24 (s, $OCH_3$), 4.18 (d, $J_{1',2'}$=7.5Hz, $H_{1'}$), 4.57 (d, $J_{4'',5''}$=10Hz, $H_{4''}$), 5.03 (broad d, $J_{1'',2''ax}$=3Hz, $H_{1''}$), 5.12 (broad, d, $J_{2a,3}$=10Hz, $H_3$), 5.72 (d, $J_{13,14}$=10.5Hz, $H_{13}$), 6.23 (d, $J_{10,11}$=16Hz, $H_{10}$), 7.35 (d, $J_{10,11}$=16Hz, $H_{11}$).

Example 6

2'-acetyl-4"-isovaleryl YT-3927

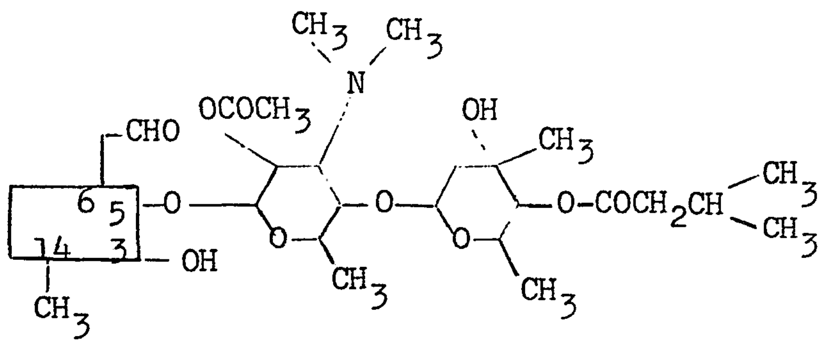

300 mg of 2'-acetyl TY-3927 (see Example 2) was dissolved in 4 ml of pyridine, and 4 ml of isovaleric anhydride was added. The mixture was stirred at 8°C for 2 days. The reaction mixture was poured into 50 ml of ice-water, and extracted with 100 ml of toluene. The extract was washed with 50 ml of a saturated aqueous solution of sodium bicarbonate twice and then with 50 ml of a saturated aqueous solution of sodium chloride, and then dried over anhydrous sodium sulfate. The sodium sulfate was removed by decantation, and the toluene was removed under reduced pressure. The residue was purified by silica gel column chromatography (Ø1.4 cm x 40 cm, benzene-acetone=8:1) to give 125 mg of the title compound having the following physicochemical properties.

$(\alpha)_D^{23}$: -70.8° (c=0.45, $CH_3OH$)

m.p.: 105 - 110°C

U.V: $\lambda_{max}^{CH_3OH}$ 2.83 nm, $E_{1cm}^{1\%}$ 239

I.R. (KBr tablet, $cm^{-1}$):

3500, 2960, 2720, 1737, 1680, 1590, 1230

NMR (Same as in Example 2) $\delta_{ppm}$:

0.97 (d, J=6Hz, $-CH(CH_3)_2$), 1.78 (s, 12-$CH_3$), 2.39 (s, $N(CH_3)_2$), 4.56 (d, $J_{4'',5''}$=10Hz, $H_{4''}$), 3.77 (broad, d, $J_{2a,3}$=10Hz, $H_3$), 5.60 (d, $J_{13,14}$=10.5Hz, $H_{13}$), 6.24 (d, $J_{10,11}$=16Hz, $H_{10}$), 7.24 (d, $J_{10,11}$=16Hz, $H_{11}$), 9.63 (s, CHO).

Example 7

4"-isovaleryl YT-3927

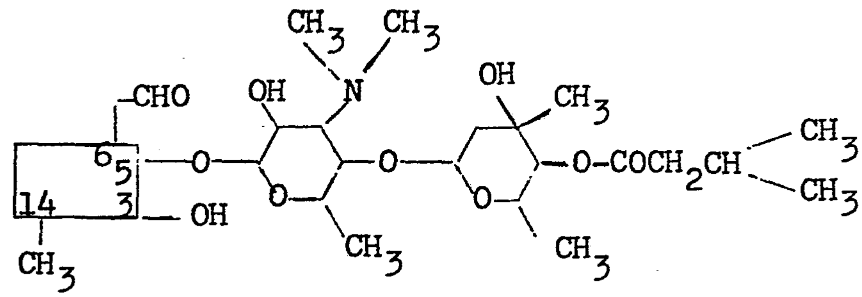

110 mg of 2'-acetyl-4"-isovaleryl YT-3927 (see Example 6) was dissolved in 30 ml of methanol, and the solution was heated under reflux for 15 hours. After cooling, methanol was removed under reduced pressure. The residue was purified by silica gel column chromatography (Ø1.4 cm x 10 cm, benzene-acetone=4:1) to give 84 mg of the title compound having the following physicochemical properties.

$(\alpha)_D^{23}$:-55.1° (c=0.69, $CH_3OH$)

m. p.:103 - 106°C

U.V.: $\lambda_{max}^{CH_3OH}$ 283 nm, $E_{1\,cm}^{1\%}$ 250

I.R. (KBr tablet, $cm^{-1}$):

3442, 2960, 2930, 2720, 1725, 1680, 1590

NMR (Same as in Example 2) $\delta_{ppm}$:

0.98 (d, J=6Hz, $COCH_2CH(C\underline{H}_3)_2$), 1.79 (s), 2.50 (s), 4.20 (d), 4.59 (d), 5.62 (d), 6.22 (d), 7.25 (d), 9.66 (s).

Example 8

3,2',3",4"-tetraacetyl YT-3927 dimethyl acetal

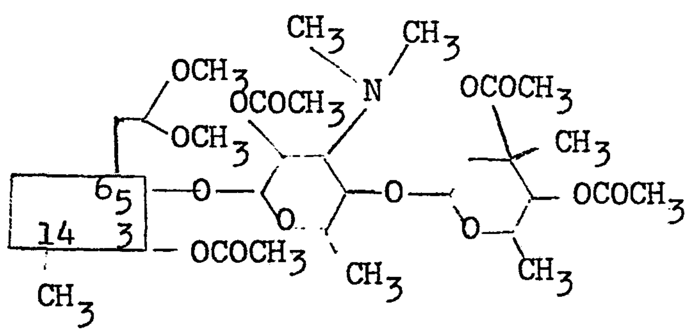

524 mg of 3,2',4"-triacetyl YT-3927 dimethyl acetal (see Example 5) and 71.25 mg of 4-dimethylaminopyridine were dissolved in 10 ml of benzene, and 2.21 ml of acetic anhydride and 3.23 ml of triethylamine were added. The mixture was heated at 70°C for 20 hours with stirring. After cooling, the reaction mixture was poured into 100 ml of toluene, and the toluene layer was washed with 50 ml of a saturated aqueous solution of sodium bicarbonate twice and then with 50 ml of a saturated aqueous solution of sodium chloride, and then dried over anhydrous sodium sulfate. The dried solution was filtered, and the solvent was removed under reduced pressure. The residue was purified by silica gel column chromatography (Ø1.4 cm x 40 cm, benzene/acetone=8/1) to give 334 mg of the title compound having the following physicochemical properties.

$(\alpha)_D^{23}$: -69.5° (c=1.0, $CH_3OH$)

m.p. : 98 - 101°C

U.V.: $\lambda_{max}^{CH_3OH}$ 283 nm, $E_{1\,cm}^{1\%}$ 217

I.R (KBr tablet, $cm^{-1}$):

2975, 2940, 1745, 1665, 1600, 1235

NMR (Same as in Example 2) $\delta_{ppm}$:

1.41 (s, 3"-$CH_3$), 1.78 (s, 12-$CH_3$), 2.00 (s, 3"-$OCOCH_3$), 2.05 (s, 3-$OCOCH_3$ and 2'-$OCOCH_3$), 2.42 (s), 3.20 (s, $OCH_3$), 3.24 (s, $OCH_3$), 4.77 (broad, d), 5.71 (d), 7.25 (d).

Example 9

3,3",4"-triacetyl YT-3927

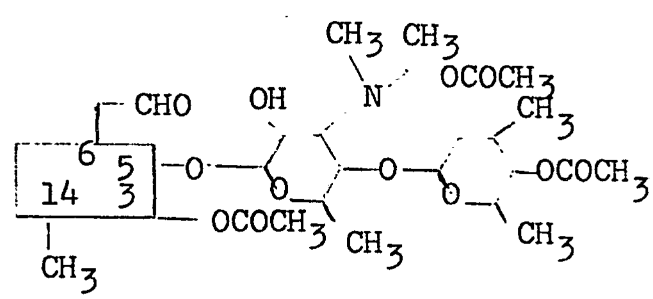

280 mg of 3,2',3",4"-tetraacetyl YT-3927 dimethyl acetal (see Example 8) was dissolved in 30 ml of methanol, and the solution was heated under reflux for 22 hours. The reaction mixture was cooled, concentrated under reduced pressure, and then dissolved in 2 ml of acetonitrile. Then, 0.05N hydrochloric acid was added, and the mixture was allowed to stand at room temperature for 2 hours. The pH of the mixture was adjusted to 7, and it was extracted with 50 ml of chloroform twice. The combined extracts were dried over anhydrous sodium sulfate, and purified by silica gel column chromatography (Ø1.4 cm x 20 cm benzene-acetone= 6:1, and 5:1) to give 124 mg of the title compound having the following physicochemical properties.

$(\alpha)_D^{23}$: -51.0° (c=1.0, $CH_3OH$)

m.p.:115 - 119°C

U.V.: $\lambda_{max}^{CH_3OH}$ 283 nm, $E_{1cm}^{1\%}$ 230

I.R. (KBr tablet, $cm^{-1}$):

2970, 2935, 2730, 1740, 1680, 1596, 1235

NMR (Same as in Example 2) $\delta_{ppm}$:

1.41 (s, 3"-$CH_3$), 1.79 (s, 12-$CH_3$), 1.99 (s, 3"-$OCOCH_3$), 2.10 (s, 3-$OCOCH_3$), 2.13 (s, 4"-$OCOCH_3$), 2.54 (s), 4.13 (d), 4.84 (broad, d), 5.12 (broad, d), 5.72 (d), 6.24 (d), 7.35 (d), 9.58 (s)

Example 10

2'-acetyl-4"-phenylacetyl YT-3927

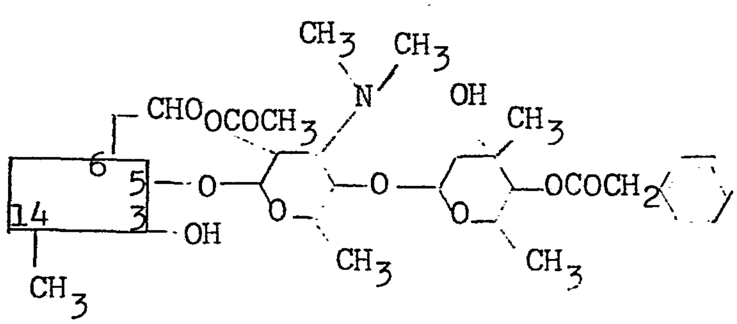

300 mg of 2'-acetyl YT-3927 (see Example 2) was dissolved in 4.8 ml of pyridine, and a solution of 0.3 ml of

phenylacetyl chloride in 4.8 ml of acetone was added at -10 to -20°C. The mixture was stirred for 30 minutes. The reaction mixture was poured into 30 ml of ice-water, and extracted with 20 ml of chloroform twice. The combined extracts were washed with 20 ml of a saturated aqueous solution of sodium bicarbonate twice and then with 20 ml of a saturated aqueous solution of sodium chloride three times, and dried over anhydrous sodium sulfate. The dried solution was filtered, concentrated, and purified by silica gel column chromatography (Ø1.4 cm x 35 cm, benzene/acetone= 8/1) to give 153 mg of the title compound having the following physicochemical properties.

$(\alpha)_D^{23}$: -59.3° (c=0.93, $CH_3OH$)

m.p.: 114 - 116°C

U.V.: $\lambda_{max}^{CH_3OH}$ 283 nm, $E_{1cm}^{1\%}$ 250

I.R. (KBr tablet, $cm^{-1}$):

3500, 2965, 1717, 1593, 1230

NMR (Same as in Example 2) $\delta_{ppm}$

1.77(s), 2.05 (s), 2.36 (s), 3.65 (s, -$C\underline{H}_2$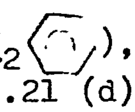), 4.24 (d), 4.51 (d), 5.60 (d), 6.22 (d), 7.21 (d), 7.25 (m, aromatic), 9.62 (s)

Example 11

4"-phenylacetyl YT-3927

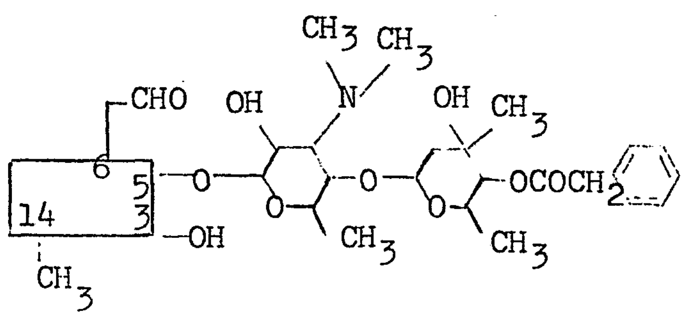

153 mg of 2'-acetyl-4'-phenylacetyl YT-3927 (see Example 10) was hydrolyzed in the same way as in Example 6, and then worked up in the same way as in Example 6 to give 57 mg of the title compound having the following physicochemical properties.

$(\alpha)_D^{23}$:-74.5° (c=0.93, $CH_3OH$)

m.p.: 127.5 - 128.5°C

U.V.: $\lambda_{max}^{CH_3OH}$ 284 nm, $E_{1cm}^{1\%}$ 273

I.R. (KBr tablet, $cm^{-1}$):

3475, 2960, 2925, 1720, 1675, 1590

NMR (Same as in Example 2) $\delta_{ppm}$:

1.79 (s), 2.48 (s), 3.77 (s, -C$\underline{H}_2$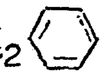),

5.62 (d), 6.24 (d), 7.31 (m, aromatic and $H_{11}$),

9.65 (s).

Example 12

20,2',3"-triacetyl-3,20-O-cyclo-4"-(4-pyridylthio)-acetyl YT-3927

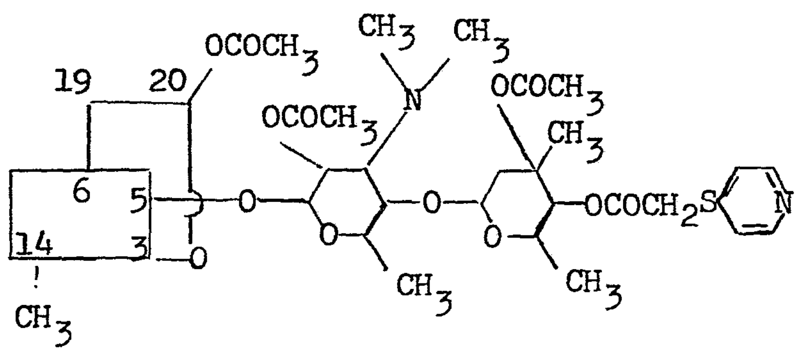

2.66 g of (4-pyridylthio)acetic acid was suspended in 50 ml of dichloromethane, and 1.59 g of triethylamine and 1.90 g of pivaloyl chloride were added. The mixture was stirred at room temperature for 1 hour. The reaction mixture was concentrated to dryness under reduced pressure, and suspended in 20 ml of benzene. Furthermore, 2.0 g of 20,2',4"-triacetyl-3,20-O-cyclo YT-3927 (see Example 3), 1.59 g of triethylamine and 287 mg of 4-dimethylaminopyridine were added, and the mixture was heated under reflux for 24 hours. After cooling, the reaction mixture was poured into 100 ml of benzene, followed by washing with a saturated aqueous solution of sodium bicarbonate and a saturated aqueous solution of sodium chloride and dried over anhydrous sodium sulfate. The dried product was filtered, and concentrated under reduced pressure. The residue was purified by silica gel column chromatography (Ø1.7 cm x 50 cm, benzene/acetone=8:1 and 6:1) to give 554 mg of the title compound

having the following physicochemical properties.

Melting point: 111 - 119°C.

U.V.: $\lambda_{max}^{CH_3OH}$ 273 nm, $E_{1cm}^{1\%}$ 275

I.R. (KBr tablet, $cm^{-1}$):

2970, 2930, 2880, 1740, 1655, 1620, 1600, 1580, 1240

NMR (Same as in Example 2) $\delta_{ppm}$:

1.37 (s, 3"-$CH_3$), 1.89 (s, 3"-$OCOCH_3$), 1.90 (s), 1.92 (s, 20-$OCOCH_3$), 2.34 (s), 3.78 (s, $COCH_2S$-), 4.10 (d), 4.53 (d), 4.75 (broad, d), 5.80 (broad, d), 6.01 (d), 6.20 (d), 6.93 (d), 7.18 (m, pyridine-β), 8.37 (m, pyridine α).

Example 13

3"-acetyl-4"-(4-pyridylthio)acetyl YT-3927

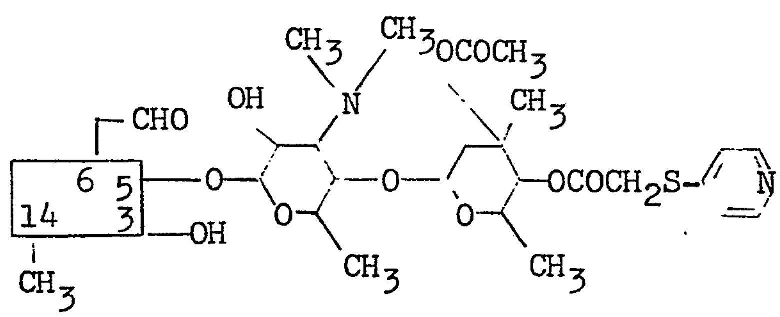

554 mg of 20,2',3"-triacetyl-3,20-O-cyclo-4"-(4-pyridylthio)acetyl YT-3927 (see Example 12) was dissolved in a small amount of acetonitrile, and 20 ml of 0.05N hydrochloric acid was added. The mixture was stirred at room temperature for 15 hours. Then, the reaction mixture was made weakly alkaline, and extracted with benzene. The extract was concentrated under reduced pressure to remove benzene. The residue was dissolved in 20 ml of methanol, and the solution was heated under reflux for 15 hours. The reaction mixture was cooled and concentrated under reduced pressure to remove methanol. The residue was purified by Sephadex LH-20 column chromatography (Ø1.4 cm x 50 cm, methanol) to give 235 mg of the title compound having the following physicochemical properties.

0052361

m.p.: 111.5 - 119.5°C

U.V.: $\lambda_{max}^{CH_3OH}$ 275 nm ($E_{1cm}^{1\%}$ 243)

I.R. (KBr tablet, $cm^{-1}$):

2960, 2920, 2870, 2720, 1730, 1675, 1585, 1240

NMR (Same as in Example 2) $\delta_{ppm}$:

1.37 (s), 1.75 (s), 1.95 (s), 2.51 (s), 3.77 (s, $-COCH_2S-$), 4.16 (d), 4.54 (d), 4.80 (broad, d), 5.60 (d), 6.20 (d), 7.22 (m, pyridine-β), 7.24 (d), 8.36 (m, pyridine-α), 9.62 (s, CHO).

Example 14

2'-acetyl-4"-(α-chloroacetoxy)phenylacetyl YT-3927

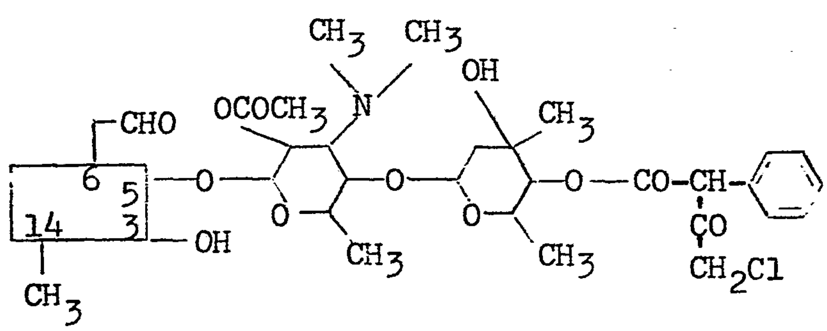

One gram of 2'-acetyl YT-3927 (see Example 3), 300 mg of α-chloroacetylmandelic acid chloride, 50 ml of benzene and 1 ml of pyridine were mixed, and stirred at 5°C for 3 hours. The reaction mixture was poured into 100 ml of benzene, washed with a saturated aqueous solution of sodium bicarbonate and a saturated aqueous solution of sodium chloride, and dried over anhydrous sodium sulfate. The dried solution was filtered, and the benzene was removed under reduced pressure. The residue was purified by silica gel column chromatography (Ø1.7 cm x 40 cm, benzene-acetone=8:1) to give 150 mg of the title compound having the following physicochemical properties.

$[\alpha]_D^{23}$: -57.1° (c 1.0, $CH_3OH$)

m.p.: 116 - 123°C

U.V.: $\lambda_{max}^{CH_3OH}$ 283 nm, $E_{1cm}^{1\%}$ 187

I.R. (KBr tablet, $cm^{-1}$):

2960, 2930, 1745, 1720, 1680, 1590, 1230, 800

NMR (Same as in Example 2) $\delta_{ppm}$:

1.79 (s), 2.05 (s, 2'$OCOCH_3$), 2.40 (s), 3.75 (broad, d), 4.19 (s, $OCOCH_2Cl$), 4.51 (d), 4.67 (m), 5.00 (broad, d), 5.60 (d), 5.93 (s, 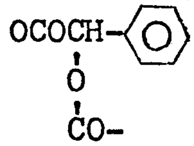), 6.24 (d), 7.22 (d), 7.33 (m, aromatic), 9.61 (s).

Example 15

4"-(α-hydroxy)phenylacetyl YT-3927

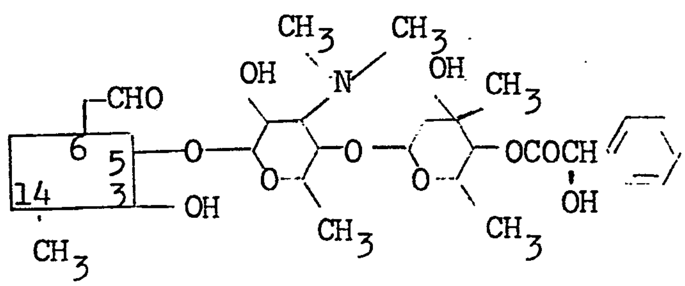

150 mg of 2'-acetyl-4"-(α-chloroacetoxy)phenylacetyl YT-3927 (see Example 14) was treated in the same way as in Example 7 to give 72 mg of the title compound having the following physicochemical properties.

$(\alpha)_D^{23}$: $-52.8^o$ (c=0.1, $CH_3OH$)

U.V.: $\lambda_{max}^{CH_3OH}$ 283 nm, $E_{1cm}^{1\%}$ 236

I.R. (KBr tablet, $cm^{-1}$):

2950, 2920, 1715, 1670, 1580

NMR (Same as in Example 2) $\delta_{ppm}$:

1.77 (s), 2.74 (s), 4.53 (d), 4.65 (m), 5.05 (broad, d), 5.28 (s, 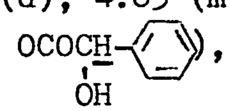), 5.61 (d), 6.22 (d), 7.24 (d), 9.61 (s).

Example 16

2'-acetyl-4"-cyclohexylcarbonyl YT-3927

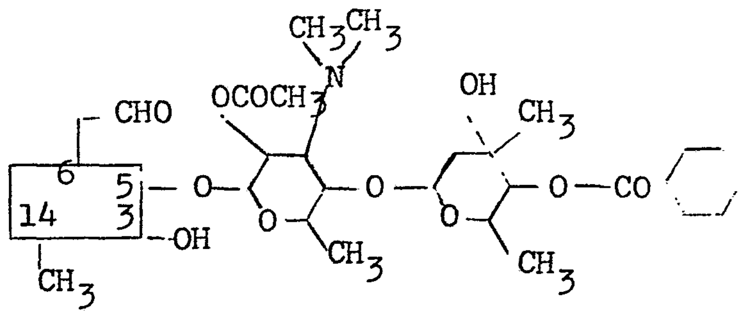

300 mg of 2'-acetyl YT-3927 (see Example 2) was dissolved in 10 ml of benzene, and 12 ml of pyridine and 9 g of cyclohexane-carboxylic anhydride were added. The mixture was stirred at room temperature for 6 days. The reaction mixture was poured into 100 ml of water, and extracted with 50 ml of chloroform twice. The combined extracts were washed with 50 ml of water and 50 ml of a saturated aqueous solution of sodium chloride twice, and dried over anhydrous sodium sulfate. The dried solution was filtered and concentrated, and the residue was purified by silica gel column chromatography (Ø1.7 cm x 20 cm, benzene-acetone=11:1) to give 77 mg of the title compound having the following physicochemical properties.

$(\alpha)_D^{23}$: -73.1° (c=0.69, $CH_3OH$)

m.p.: 112 - 115°C

U.V.: $\lambda_{max}^{CH_3OH}$ 284 nm, $E_{1cm}^{1\%}$ 226

I.R. (KBr tablet, $cm^{-1}$):

3490, 2950, 2920, 1740, 1720, 1675, 1585, 1225

NMR (Same as in Example 2) $\delta_{ppm}$:

1.76 (s), 2.04 (s), 2.38 (s), 5.00 (broad, d), 5.60 (d), 6.24 (d), 7.24 (d), 9.64 (s).

Example 17

4"-cyclohexylcarbonyl YT-3927

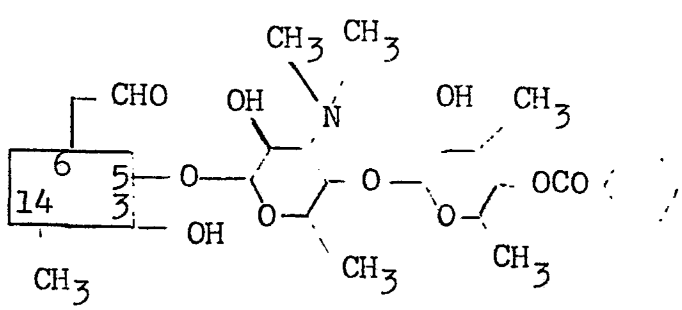

77 mg of 2'-acetyl-4"-cyclohexylcarbonyl YT-3927 (see Example 16) was treated in the same way as in Example 6 to give 48 mg of the title compound having the following physicochemical properties.

$(\alpha)_D^{23}$: -60.0° (c=0.75, $CH_3OH$)

m.p.: 120 - 122°C

U.V.: $\lambda_{max}^{CH_3OH}$ 284 nm, $E_{1cm}^{1\%}$ 254

I.R. (KBr tablet, $cm^{-1}$):

3490, 2950, 2920, 1720, 1680, 1590

NMR (same as in Example 2) $\delta_{ppm}$:

1.78 (s), 2.50 (s), 4.18 (s), 5.01 (broad, d), 5.64 (d), 6.26 (d), 7.26 (d), 9.68 (s)

Example 18

2'-acetyl-4"-phenylcarbamoyl YT-3927

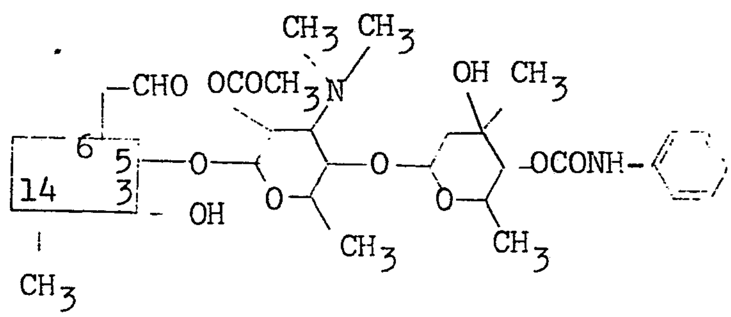

400 mg of 2'-acetyl YT-3927 (see Example 2) was dissolved in 10 ml of benzene, and 238 mg of phenyl isocyanate and 2 drops of pyridine were added. The mixture was stirred at room temperature for 2 hours, and then at 40 to 45°C for 43 hours. The reaction mixture was cooled, poured into 50 ml of benzene, and washed with a saturated aqueous solution of sodium chloride, followed by drying over anhydrous sodium sulfate. The dried solution was filtered, and then dried under reduced pressure. The residue was purified by silica gel column chromatography (Ø1.7 cm x 40 cm, benzene-acetone=8:1) to give 141 mg of the title compound having the following physicochemical properties.

m.p. 120 - 149°C

U.V.: $\lambda_{max}^{CH_3OH}$ 283, and 236 nm

Example 19

4"-phenylcarbamoyl YT-3927

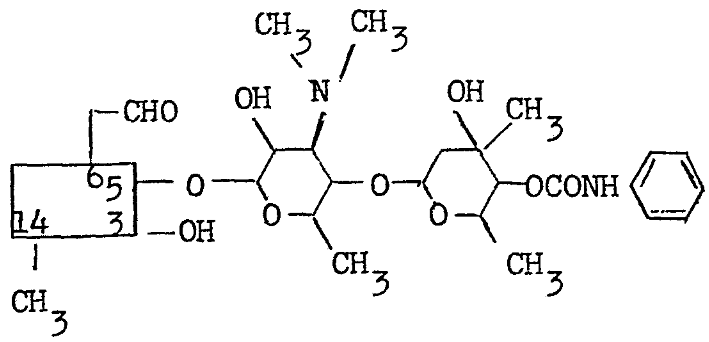

140 mg of 2'-acetyl-4"-phenylcarbamoyl YT-3927 (see Example 18) was treated in the same way as in Example 7 to give 115 mg of the title compound having the following physicochemical properties.

Melting point: 125 - 148°C

U.V.: $\lambda_{max}^{CH_3OH}$ 236 nm ($E_{1cm}^{1\%}$ 252),

$\lambda_{max}^{CH_3OH}$ 283 nm ($E_{1cm}^{1\%}$ 252)

I.R. (KBr tablet, $cm^{-1}$):

2960, 2930, 2730, 1720, 1680, 1595, 755

N.M.R (same as in Example 2) $\delta_{ppm}$:

1.77 (s), 2.48 (s), 4.18 (d), 4.51 (d), 5.04 (broad, d), 5.61 (d), 6.20 (d), 6.85 - 7.43 (m, aromatic), 7.23 (d), 9.62 (s).

Example 20

2'-acetyl-4"-tosyl YT-3927

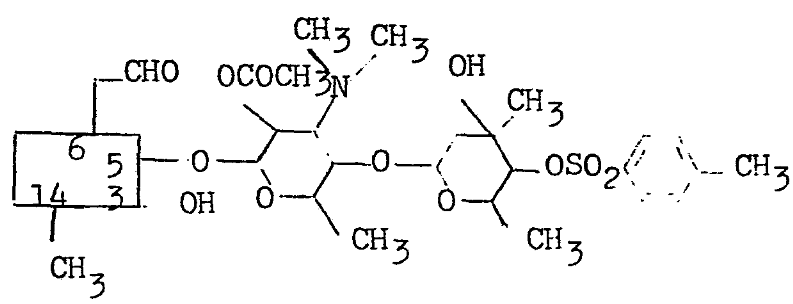

300 mg of 2'-acetyl YT-3927 (see Example 2) and 744 mg of tosyl chloride were dissolved in 5 ml of pyridine, and the solution was stirred at 8°C for 18 hours. The reaction mixture was poured into 50 ml of ice water to

decompose the excess of tosyl chloride, and then extracted with 100 ml of toluene. The extract was washed with 50 ml of a saturated aqueous solution of sodium bicarbonate twice, and then with 50 ml of an aqueous solution of sodium chloride, and then dried over anhydrous sodium sulfate. The anhydrous sodium sulfate was removed by decantation, and the solvent was removed under reduced pressure. The residue was purified by silica gel column chromatography (Ø1.7 cm x 50 cm, benzene-acetone=8:1 and 7:1) to give 216 mg of the title compound having the following physicochemical properties.

$(\alpha)_D^{23}$: -69.7° (c=1.0, $CH_3OH$)
m.p.: 129 - 131°C
U.V.: $\lambda_{max}^{CH_3OH}$ 283 nm ($E_{1cm}^{1\%}$ 231),
$\lambda_{max}^{CH_3OH}$ 226 nm ($E_{1cm}^{1\%}$ 166)

Example 21

4"-tosyl YT-3927

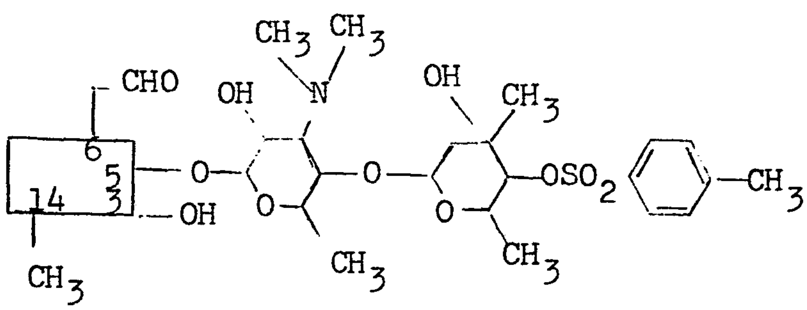

190 mg of 2'-acetyl-4"-tosyl YT-3927 (see Example 20) was treated in the same way as in Example 6 to give 176 mg of the title compound having the following physicochemical properties.

$(\alpha)_D^{23}$: -53.4° (c=1.0, $CH_3OH$)
m.p.: 123 - 127°C
U.V.: $\lambda_{max}^{CH_3OH}$ 283 nm ($E_{1cm}^{1\%}$ 237),
$\lambda_{max}^{CH_3OH}$ 226 nm ($E_{1cm}^{1\%}$ 171)
I.R. (KBr tablet, $cm^{-1}$):
2970, 2940, 2730, 1720, 1680, 1595, 1180

NMR (same as in Example 2) $\delta_{ppm}$:
1.82 (s), 2.41 (s, $-\langle\rangle-C\underline{H}_3$), 2.44 (s), 4.17 (d), 4.26 (d), 4.68 (m), 4.99 (broad, d), 5.62 (d), 6.22 (d), 7.27 and 7.77 (ABq, aromatic), 9.65 (s).

Example 22

2'-acetyl-4"-tetrahydropyranyl YT-3927

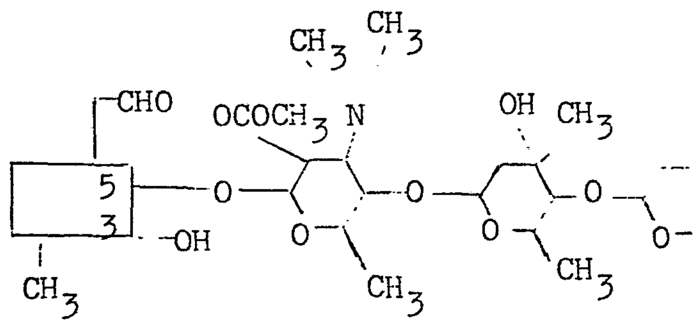

266 mg of 2'-acetyl YT-3927 (see Example 2) was dissolved in 5 ml of dichloromethane, and the solution was cooled to -10 to -20°C. To the solution were added 0.44 ml of 3,4-dihydropyrane and 0.039 ml of trifluoroacetic acid. The mixture was stirred at room temperature for 24 hours. The reaction mixture was poured into 50 ml of ice water and extracted with 60 ml of benzene. The extract was washed with 30 ml of a saturated aqueous solution of sodium bicarbonate and 30 ml of a saturated aqueous solution of sodium chloride, and dried over anhydrous sodium sulfate. The dried solution was filtered and concentrated. The residue was purified by silica gel column chromatography (Ø1.7 cm x 22 cm, benzene-acetone=8:1) to give 101 mg of the title compound having the following physicochemical properties.

$[\alpha]_D^{23}$: -70.7° (c=0.64, $CH_3OH$)
m.p.: 111 - 114°C

Example 23

4"-tetrahydropyranyl YT-3927

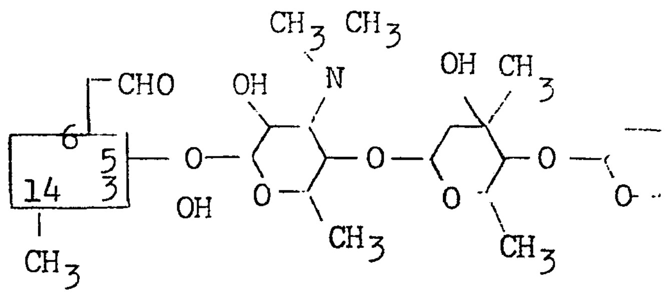

141 mg of 2'-acetyl-4"-tetrahydropyranyl YT-3927 (see Example 22) was dissolved in 30 ml of methanol, and the solution was heated under reflux for 9 hours. The reaction mixture was cooled, and then methanol was removed under reduced pressure. The residue was purified by silica gel column chromatography (Ø1.4 cm x 7 cm, benzene-acetone=6:1) to give 40.2 mg of the title compound having the following physicochemical properties.

$(\alpha)_D^{23}$: -62.4° (c=0.69, $CH_3OH$)

m.p.: 116 - 120°C

U.V.: $\lambda_{max}^{CH_3OH}$ 284 nm, $E_{1cm}^{1\%}$ 265

I.R. (KBr tablet, $cm^{-1}$):

3480, 2950, 2920, 1715, 1675, 1585

NMR (same as in Example 2) $\delta_{ppm}$:

1.76 (s), 2.50 (s), 4.16 (d), 4.95 (broad, d), 5.54 (d), 6.21 (d), 7.22 (d), 9.64 (s).

Example 24

3-acetyl-4"-isovaleryl YT-3927

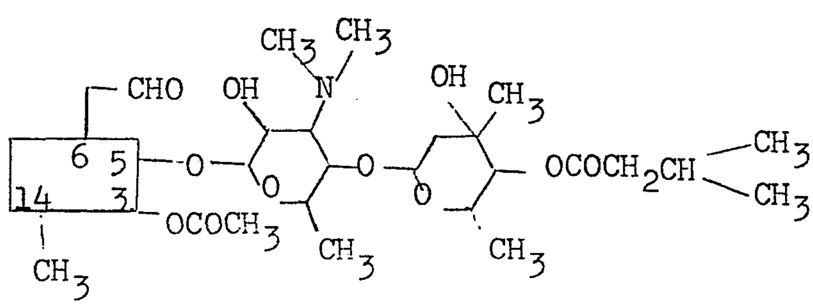

300 mg of 2'-acetyl-4"-isovaleryl YT-3927 (see Example 6) was suspended in 2 ml of pyridine, and 2 ml of acetic anhydride was added. The mixture was stirred at 20°C for 2 days. The reaction mixture was poured into 50 ml of ice-water to decompose the excessive acetic anhydride completely. The pH of the mixture was adjusted to 7, and it was extracted with 50 ml of toluene. The

toluene layer was washed with 50 ml of a saturated aqueous solution of sodium bicarbonate twice and then with 50 ml of a saturated aqueous solution of sodium chloride, and then dried over anhydrous sodium sulfate. After removal of the solvent, the residue was dissolved in 20 ml of methanol, and hydrolyzed in the same way as in Example 7 and further treated in the same way as in Example 7 to give 110 mg of the title compound having the following physicochemical properties.

m.p.: 103 - 109°C

I.R. (KBr tablet, $cm^{-1}$):

3480, 2970, 2930, 2730, 1725, 1680, 1590, 1240

NMR (same as in Example 2) $\delta_{ppm}$:

0.98 (d), 1.80 (s), 2.10 (s), 2.50 (s), 4.13 (d), 4.58 (d), 5.04 (broad, d), 5.12 (broad, d), 5.73 (d), 6.19 (d), 7.33 (d), 9.60 (s).

WHAT WE CLAIM IS:

1. A compound of the general formula

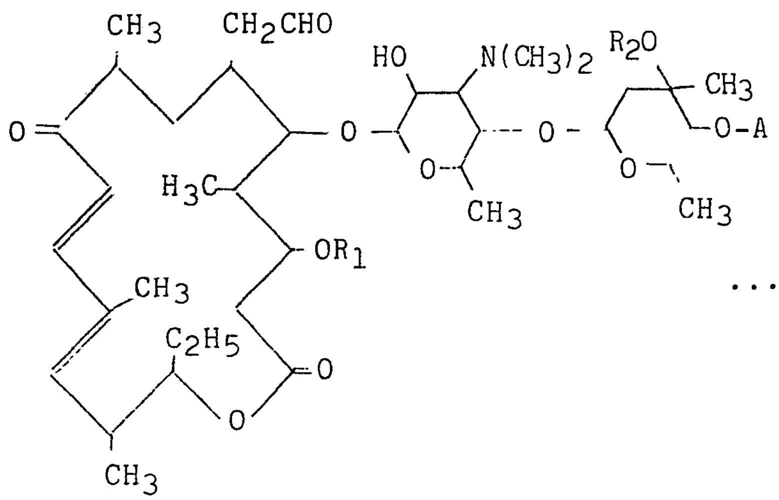

..... (I)

wherein $R_1$ and $R_2$, independently from each other, represent a hydrogen atom or a lower alkanoyl group; and A represents a hydrogen atom or a group of the formula $-Y-R_3$ in which $R_3$ represents an alkyl group, a cycloalkyl group, an aryl group, an aralkyl group, a 4- to 7-membered monovalent heterocyclic group containing 1 to 4 hetero atoms selected from nitrogen, oxygen and sulfur atoms or a group of the formula $-R_4-Z-R_5$ in which $R_4$ represents a lower alkylene group which may be substituted by a hydroxyl or lower alkanoyloxy group, $R_5$ represents an alkyl group, a cycloalkyl group, an aryl group, an aralkyl group, or a 4- to 7-membered monovalent heterocyclic group containing 1 to 4 hetero atoms selected from nitrogen, oxygen and fulfur atoms and Z represents an oxygen or sulfur atom, and Y represents -CO-, $-SO_2-$, or -CONH-, or Y and $R_3$, taken together, represent a tetrahydrofuranyl or tetrahydropyranyl group; and the aforesaid aryl group, aralkyl group and heterocyclic group may be substituted, as required, by a lower alkyl, lower alkoxy or nitro group;

or its salt.

2. The compound of claim 1 wherein $R_1$ and $R_2$ each represent a hydrogen atom or an acetyl group.

3. The compound of claim 1 or 2 wherein A represents a hydrogen atom.

4. The compound of claim 1 wherein A represents a group of the formula $-Y-R_3$ in which Y represents -CO-, and $R_3$ represents a group of the following formula

$$-\underset{R_6}{\underset{|}{CH}}-Q-R_7$$

wherein $R_6$ represents a hydrogen atom, a hydroxyl group or an acetyloxy group, $R_7$ represents a lower alkyl group, a phenyl group which may be substituted by one methyl or nitro group, or a pyridyl group, and Q represents a direct bond or an oxygen or sulfur atom.

5. A compound of the formula

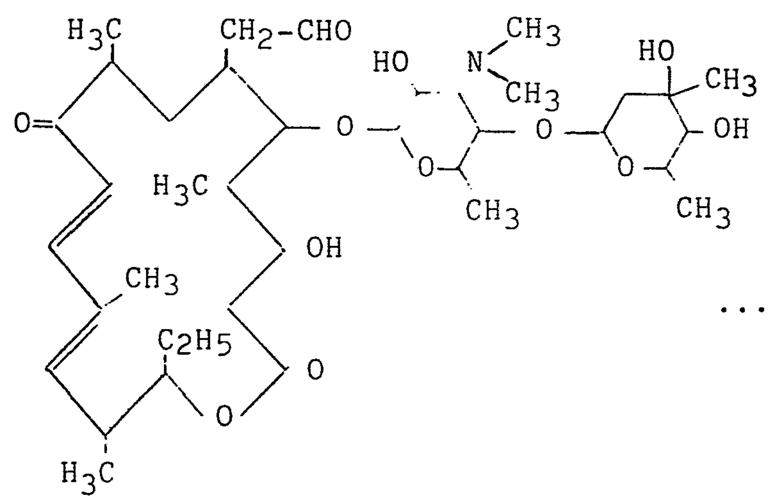

..... (I-a)

or its salt.

6. A pharmaceutical composition comprising the compound of any one of claims 1 to 5 or its pharmaceutically acceptable salt in admixture with a pharmaceutically acceptable carrier or diluent.

7. Use of the compound of any one of claims 1 to 6 or its pharmaceutically acceptable salt for the prevention, therapy or treatment of bacterial infections of mammals.

0052361

European Patent Office

**PARTIAL EUROPEAN SEARCH REPORT**
which under Rule 45 of the European Patent Convention shall be considered, for the purposes of subsequent proceedings, as the European search report

Application number
EP 81 10 9685

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| Y | 2321 THE JOURNAL OF ANTIBIOTICS, vol. XXXIII, Aug. 1980, no. 8, Tokyo, JP<br>S. OMURA: "Production of mycarosyl protylonolide by a mycaminose idiotroph from the tylosin-producing strain"Streptomyces Fradiae KA-427, pages 913-914<br>* Pages 913-914 *<br>-- | 1,6 | C 07 H 17/08<br>A 61 K 31/70<br>C 12 P 19/62//<br>(C 12 P 19/62<br>C 12 R 1/54) |
| Y | THE JOURNAL OF ANTIBIOTICS, vol. XXIX, no. 11<br>An International Journal Devoted to Research on Antibiotics and Other Microbial Products<br>TOYOKAZU KISHI et al.: "Studies on juvenimicin, a new antibiotic. II Isolation, chemical characterization and structures"pages 1171-81<br>* Page 1176: Juvenimicin B *<br>-- | 1,6 | TECHNICAL FIELDS SEARCHED (Int. Cl.3)<br>C 07 H 17/00<br>A 61 K 31/00<br>C 12 P 19/00 |
| Y | THE JOURNAL OF ORGANIC CHEMISTRY, vol. 44, no. 12, June 8, 1979 pages 20-50-2052<br>A. NAGEL: "Selective Cleavage of the Mycinose Sugar from the ../. | | |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-6
Claims searched incompletely:
Claims not searched: 7 Method for treatment of the
Reason for the limitation of the search: human or animal body by surgery or therapy (See article 52(4) of the European Patent Convention)

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| Place of search | Date of completion of the search | Examiner |
|---|---|---|
| The Hague | 17-02-1982 | VERHULST |

EPO Form 1505.1 06.78

0052361

European Patent Office

# PARTIAL EUROPEAN SEARCH REPORT

Application number

EP 81 10 9685

-2-

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | Macrolide Antibiotic Tylosin: A Unique Glycosidic Scission"<br>* Page 2050: formula 3 *<br>---- | 1,6 | |
| | | | TECHNICAL FIELDS SEARCHED (Int. Cl.3) |

EPO Form 1505.3 06.78